# EUROPEAN PATENT APPLICATION

(11) **EP 4 663 240 A1**
(43) Date of publication of application: **17.12.2025**
(21) Application number: 24181738.6
(22) Date of filing: 12.06.2024
(51) Int. Cl.: A61P 21/00, C07K 16/24

(54) **IFN- PATHWAY INHIBITORS FOR USE IN THE TREATMENT OF FSHD**

(71) Applicant: Ospedale San Raffaele S.r.l., 20132 Milano (IT)
(72) Inventor: Gabellini, Davide, 20132 Milan (IT); Biferali, Beatrice, 20132 Milan (IT)
(74) Representative: Pace Napoleone, Maria

(57) **Abstract**

The present invention provides inhibitors of IFN-γ signaling reducing the inflammatory insult caused by aberrant DUX4 expression as a novel therapeutic approach for the treatment of FSHD.

## Description

### FIELD OF THE INVENTION

The present invention, by identifying that inhibition of IFN-γ signaling significantly reduce muscle inflammation, FAPs expansion, fibro-fatty conversion, and overall disease progression in the mouse model of facioscapulohumeral muscular dystrophy (FSHD), provides selective drugs reducing the inflammatory insult caused by aberrant DUX4 expression. These drugs provide a novel therapeutic approach for the treatment of FSHD.

### BACKGROUND

Facioscapulohumeral muscular dystrophy (FSHD), one of the most prevalent neuromuscular disorders, is a chronic disease leading to significant lifetime morbidity. FSHD indiscriminately afflicts children and adults of all ages and sexes. The disease is caused by aberrant muscle expression of double homeobox 4 (DUX4) ¹. DUX4 encodes for a transcription factor physiologically expressed during early embryonic development being involved in zygotic genome activation at the cleavage stage ^{2 3 4}. DUX4 is normally silent in skeletal muscle and its aberrant expression is associated with induction of several pathways, including oxidative stress and DNA damage, inhibition of myogenic differentiation, impaired transcript quality control and inflammation ¹.

Worryingly, there is increasing evidence that, once DUX4 has activated its target genes, the FSHD pathogenic pathways become independent from DUX4 expression though several self-sustained positive feedforward loops ⁵⁻¹⁰. The possibility that DUX4 downstream pathways might persist in the absence of DUX4 raises concern regarding the current emphasis on the development of therapeutic approaches directly targeting DUX4 expression. Therefore, the identification of pathways downstream of DUX4 that are required for the pathogenesis of FSHD bears strong therapeutic relevance. However, it is currently unclear which DUX4 downstream process is responsible for FSHD and if its manipulation leads to any therapeutic benefit. Consequently, despite several clinical trials ^{11 12 13 14 15 16 17 18 19 20 21 22}, there continues to be no cure or therapeutic treatment available to FSHD patients.

Muscle inflammation is a prominent feature that takes place early on in FSHD, is associated with active disease and anticipates the loss of muscle tissue and its fibro-fatty substitution, unlike classical muscular dystrophies in which inflammation is generally secondary to muscle wasting. FSHD muscles with signs of inflammation have a faster progression to fat replacement than those with low inflammatory signs, and the fat replacement progression correlates with the severity of inflammation ^{23 24 25 26}. Intriguingly, recent data suggest that a systemic immune response to FSHD antigens might have a role in disease progression ²⁷. Nevertheless, despite the overwhelming indication for its primary role in the disease, the nature of these "FSHD antigens" and the molecular mechanism responsible for FSHD muscle inflammation are currently unknown. Moreover, and differently from Duchenne muscular dystrophy (DMD), the anti-inflammatory drugs tested so far (mainly corticosteroids) have not shown any effect on FSHD patients ^{11 12 15 16 22}. It is tempting to speculate that the nature of the inflammatory insult in FSHD differs from that of muscular dystrophies caused by mutations in the dystrophin glycoprotein complex and should hence be treated differently.

Intriguingly, DUX4 target genes are upregulated in actively inflamed STIR-positive FSHD muscle biopsies and correlate with fat replacement ^{28 29}. The overlap between activation of DUX4 targets and inflammation has been demonstrated also in the FLExDUX4 mouse model of FSHD (see below) ³⁰.

Therefore, understanding the molecular mechanism leading to FSHD muscle inflammation and fibro-fatty replacement and determining if and how DUX4 expression produces immune attack could provide relevant therapeutic opportunities.

It has recently been reported that serum interleukin-6 (IL-6) levels are significantly associated with FSHD disease duration and severity ³¹. The identification of IL-6 as the only cytokine with a concentration following the disease severity ³¹ makes it a potent biomarker for FSHD accelerating the transition toward precision medicine. IL-6 is expressed by many cell types ³² and in skeletal muscle it is produced and released by muscle fibers in response to physical activity ³³. IL-6 has a pleiotropic activity and in physiological conditions a transient production and a short-term action of IL-6 leads to anti-inflammatory, antioxidant, and pro-myogenic actions. Instead, long-lasting increased systemic levels of IL-6 can induce pro-inflammatory, pro-oxidant and pro-fibrotic responses promoting skeletal muscle atrophy ^{34 35-38 39 40,41}. The dual nature of IL-6 action raises concern regarding its targeting since blocking IL-6 activity could inhibit its muscle pro-regeneration functions beside blocking muscle inflammation. Therefore, there is strong interest in developing ways to selectively inhibit the detrimental activities while maintaining the physiologic and pro-regenerative activities associated to inflammation.

During skeletal muscle regeneration, one of the main sources of IL-6 are Fibro-Adipogenic Progenitors (FAPs) ⁴². FAPs are a population of interstitial mesenchymal stem cells, which has been recently demonstrated essential for long-term homeostatic maintenance and repair of skeletal muscle ^{43 44}. Accumulating evidence points to FAPs as pivotal effectors to tip the balance between skeletal muscle regeneration and degeneration ^{42,43,45-49}. FAPs have been defined as bi-potent muscle-resident progenitors capable to differentiate in fibroblasts and adipocytes ^{42,45,46}. During skeletal muscle regeneration, FAPs quickly proliferate and expand, prior to muscle stem cells (MuSCs), providing a transient favorable environment to promote, paracrinally, MuSCs-mediated regeneration ^{42,47,49-51}. Intriguingly, beyond their supportive role in muscle regeneration, FAPs have been shown to be the major source of fibroblasts and adipocytes in degenerating muscles ⁴⁵⁻⁴⁸. When regeneration fails, as in DMD muscles at advanced stages of disease, FAPs turn into fibro-adipocytes, being no longer able to promote MuSCs myogenic potential and leading to gradual accumulation of fatty and fibrotic infiltrate within the muscle ⁴⁶⁻⁴⁹.

Although various intracellular signaling pathways are activated after IL-6 stimulation, it is now established that the main positive signal of IL-6 is transduced by the activation of signal transducer and activator of transcription (STAT) STAT-3. Once phosphorylated, STAT-3 dissociates from the receptor complex, forms a homo- or heterodimer with STAT-1, translocates into the nucleus where it binds to DNA and activates the transcription of downstream gene targets. Notably, STAT-3 signaling contributes to muscle wasting in inflammatory myopathies, muscular dystrophies, and cancer cachexia ⁵².

In denervating muscle, a progressive accumulation of FAPs, a persistent activation of STAT-3 by elevated levels of IL-6, which promotes muscle atrophy and fibrosis, has been described ⁵³. Similarly, in FSHD patients and a FSHD mouse model, an expansion of FAPs number together with an alteration in their phenotype possibly contributing to disease severity was recently reported ^{54,55}. Notably, in this context, DUX4 aberrant expression is restricted to muscle fibers and DUX4 target genes are not elevated in FAPs suggesting that FAPs activation is indirect, possibly by factors released by muscle fibers expressing DUX4.

### SUMMARY OF THE INVENTION

It is an object of the invention an inhibitor of the IFN-γ inflammatory pathway for use in the treatment and/or prevention of FSHD.

Preferably, said inhibitor of the IFN-γ inflammatory pathway is an inhibitor of IFN-γ expression and/or function; still preferably, said inhibitor of the IFN-γ inflammatory pathway is selected from the group consisting of:
a) polynucleotide capable of inhibiting the expression of IFN-γ or a polynucleotide coding for said polynucleotide;
b) a polypeptide, preferably an antibody or a synthetic or recombinant derivative thereof;
c) a polynucleotide coding for said polypeptide or a functional derivative thereof;
d) a small molecule.

More preferably, the inhibitor of IFN-γ expression and/or function for use in the treatment and/or prevention of FSHD is anti-IFN-γ antibody preferably selected from Emapalumab (Gamifant), fontolizumab (Huzaf), AMG 811, AB_2687717.

It is a further object of the invention the inhibitor of IFN-γ expression and/or function for use in the treatment and/or prevention of FSHD, wherein said inhibitor is used in combination with:
- an inhibitor of IL-6 expression and/or function; and/or
- an inhibitor of STAT-3 expression and/or function; and/or
- an inhibitor of JAK kinases expression and/or function.

Preferably the inhibitor of IL-6 expression and/or function and the inhibitor of STAT-3 expression and/or function and the inhibitor of JAK kinases expression and/or function are selected from the group consisting of:
a) polynucleotide capable of inhibiting the expression of the IL-6 and/or of STAT-3 and/or JAK kinases or a polynucleotide coding for said polynucleotide;
b) a polypeptide, preferably an antibody or a synthetic or recombinant derivative thereof;
c) a polynucleotide coding for said polypeptide or a functional derivative thereof;
d) a small molecule.

Preferably said inhibitor of IL-6 expression and/or function is anti-IL-6 antibody, preferably said anti IL-6 antibody is selected from Tocilizumab, Siltuximab, Sarilumab, ziltivekimab, olokizumab (CDP6038), elsilimomab, clazakizumab (BMS-945429, ALD518), sirukumab (CNTO 136), levilimab (BCD-089).

Preferably the inhibitor of STAT-3 expression and/or function is a small molecule inhibitor, preferably said small molecule inhibitor is selected from atovaquone, WP1066, TTI-101, VVD-130850, OPB-51602, silibinin e KT-333. Still preferably the inhibitor of STAT3 expression and/or function is Danvatirsen.

Preferably the inhibitor of JAK kinases expression and/or function is selected from baricitinib (Olumiant), tofacitinib (Xeljanz), ruxolitinib (Jakavi, Opzelura), abrocitinib (Cibinqo), upadacitinib (Rinvoq), fedratinib (Inrebic).

In a preferred embodiment of the invention, the IFN-γ inflammatory pathway is initiated by overexpression of the DUX4 gene.

It is a further object of the invention the inhibitor of the IFN-γ inflammatory pathway as above defined for use in the treatment and/or prevention of FSHD, wherein said inhibitor is used in combination with a further therapeutic agent and/or a therapeutic intervention.

It is a further object of the invention a pharmaceutical composition for use in the treatment and/or prevention of FSHD said pharmaceutical composition comprising the inhibitor of the IFN-γ inflammatory pathway as above defined and at least one pharmaceutical acceptable carrier and/or vehicle.

It is a further object of the invention an in-vitro method for evaluating the risk and/or for diagnosis and/or for prognosis and/or for monitoring progression and/or for monitoring the efficacy of a therapeutic treatment and/or for screening of FSHD in a subject or in an ex vivo or in vitro system, preferably an organoid such as artificial skeletal muscle, comprising the steps of:
a) detect or measure the amount or activity of the IFN-γ or of fragments thereof or of the polynucleotide encoding said protein or its fragments in an isolated biological sample obtained from the subject, which may be optionally comprised in an ex vivo or in vitro system, and
b) comparison with a proper control.

The present invention will be described by means of non-limiting examples, referring to the following figures:

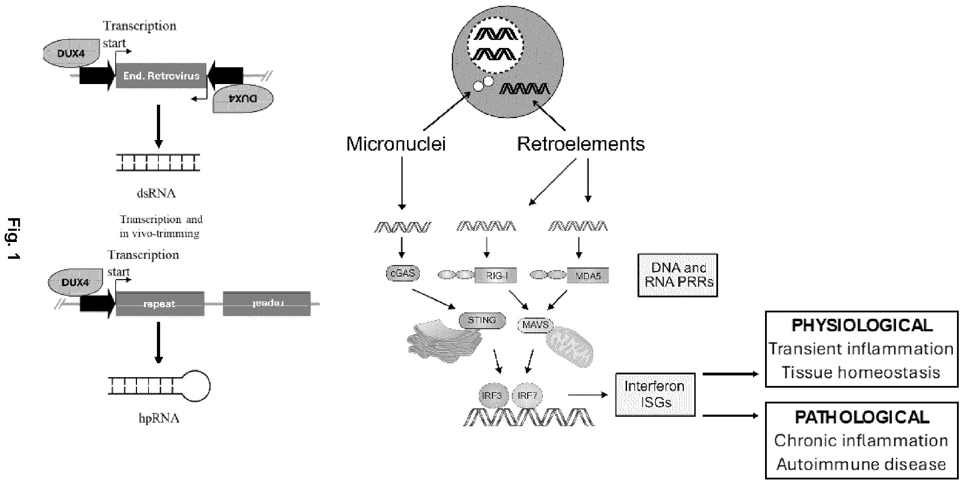

**Figure 1****. Hypothetical mechanism through which DUX4 could activate inflammation.** Left. The activation of endogenous retroviruses and other repetitive sequences by DUX4 leads to the accumulation of double strand RNA (dsRNA), which significantly contributes to DUX4 toxicity.
   Right. In other contexts, growing evidence indicates that an important consequence of dsRNA production is the induction of a "viral mimicry" status encompassing innate and adaptive immune responses and culminating with the activation of interferon signaling.
**Figure 2****. Activation of repeat RNAs and RNA sensors in FSHD.**
   Top. RNA levels of direct DUX4 target repeats evaluated by RT-qPCR in healthy donor and FSHD myotubes.
   Bottom. TLR3, MDA5 and RIG1 RNA levels evaluated by RT-qPCR in healthy donor and FSHD myotubes.
   N=3. Unpaired t-test *p<0.05; **p<0.01; ***p<0.001.
**Figure 3****. Activation of inflammatory pathway by DUX4 in FSHD.**
   Left. IFN-γ, IL-6, STAT-1 and STAT-3 RNA levels evaluated by RT-qPCR in healthy donor and FSHD myotubes.
   Center. DUX4 RNA levels evaluated by RT-qPCR in control (siNT) and DUX4 (siDUX4) knockdown FSHD myotubes.
   Right. Repeats, IFN-γ and IL-6 RNA levels evaluated by RT-qPCR in control (siNT) and DUX4 (siDUX4) knockdown FSHD myotubes.
   N=3. Unpaired t-test *p<0.05; **p<0.01.

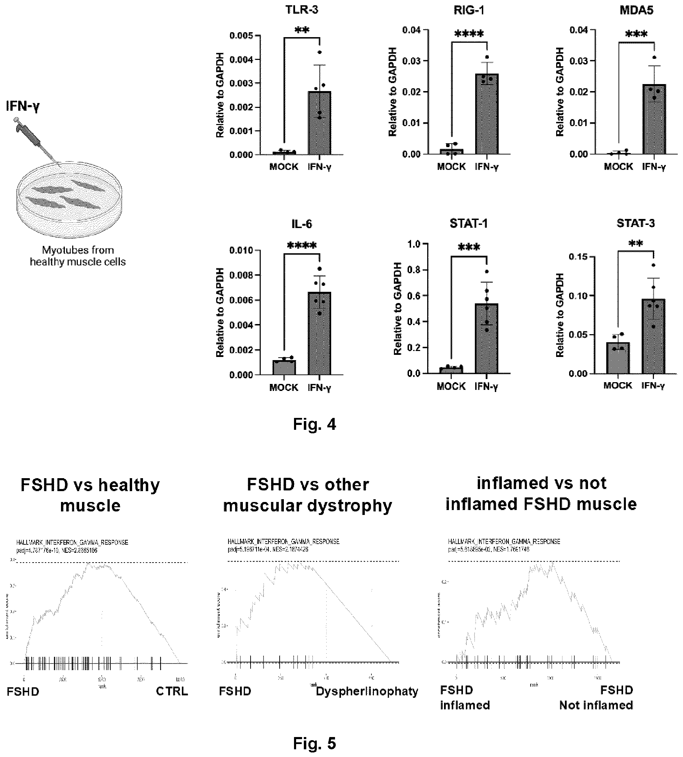

**Figure 4****. IFN-γ is sufficient to induce the viral mimicry inflammatory pathway in healthy donor muscle cells.**
   Left. Schematic representation of the IFN-γ treatment in healthy donor myotubes.
   Right. RNA sensors, IL-6, STAT-1 and STAT-3 RNA levels evaluated by RT-qPCR in vehicle (MOCK) and IFN-γ treated healthy donor myotubes.
   N=4-6. Unpaired t-test **p<0.01: ***p<0.001; ****p<0.0001.
**Figure 5****. Activation of the IFN-γ response signature in FSHD patients.**
   Publicly-available gene expression datasets using muscle biopsies from healthy donors, FSHD patients and patients affected by other muscular dystrophies used to perform gene set enrichment analysis (GSEA) on the list of genes significantly upregulated in: i. FSHD patients compared to healthy donors; ii. FSHD patients compared to patients affected by another muscular dystrophy; and iii. inflamed compared to non-inflamed muscles of FSHD patients.

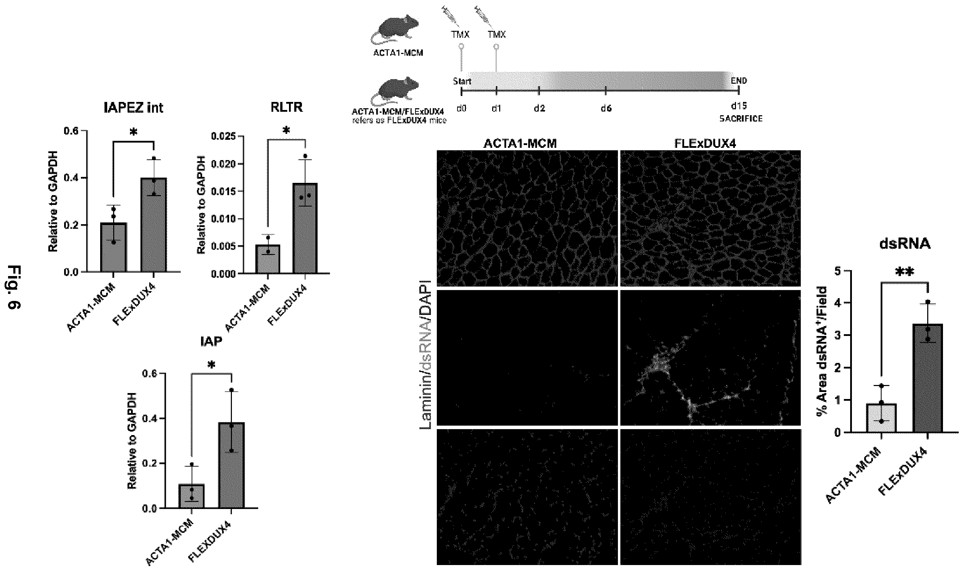

**Figure 6****. Increased repeats and dsRNA in FLExDUX4 mice.**
   Left. RNA levels of direct DUX4 target repeats evaluated by RT-qPCR in control (ACTA1-MCM) and FLExDUX4 muscle.
   Right. Staining for Laminin, dsRNA and nuclei (DAPI), and quantification of the dsRNA signal in muscle sections of control and FLExDUX4.
   N=3. Unpaired t-test *p<0.05; **p<0.01.

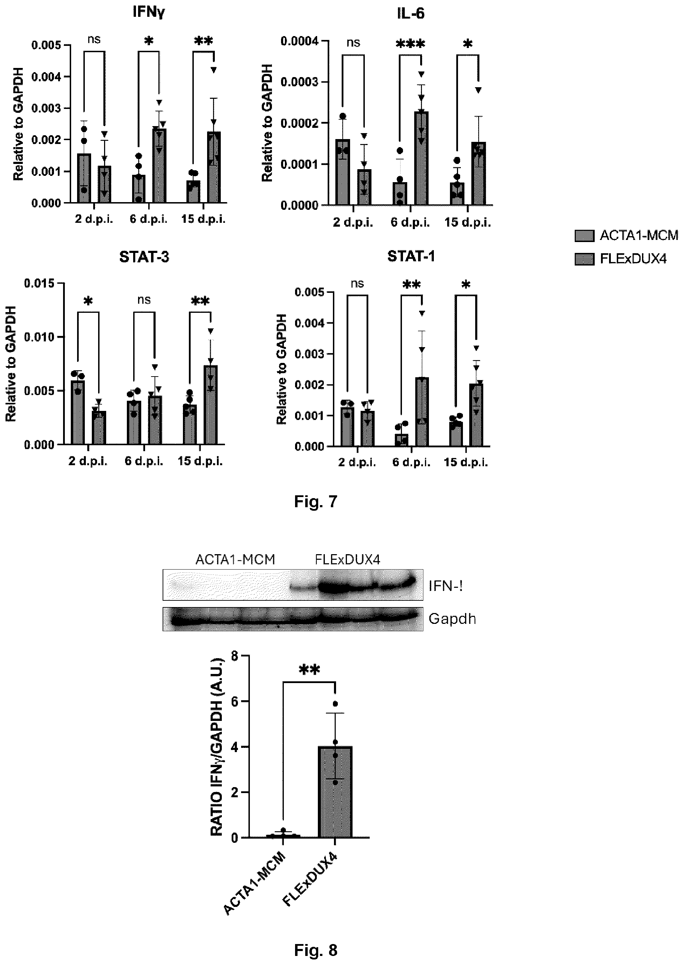

**Figure 7****. Inflammatory factors are activated in FLExDUX4 mice.**
   IFN-γ, IL-6, STAT-1 and STAT-3 RNA levels evaluated by RT-qPCR in muscle from ACTA1-MCM and FLExDUX4 mice at the indicated days post injection (d.p.i.) of tamoxifen. N=3-6. Two-way analysis of variance (ANOVA), with Šídák's multiple comparisons test, *p<0.05; **p<0.01; ***p<0.001.
**Figure 8****. Increased IFN-γ protein level in FLExDUX4 mice.**
   Top. Total muscle extracts from ACTA1-MCM and FLExDUX4 mice analyzed with IFN-γ and Gapdh antibodies, as loading control.
   Bottom. Densitometric quantification of IFN-γ signal normalized for Gapdh.
   N=4. Unpaired t-test, **p<0.01.

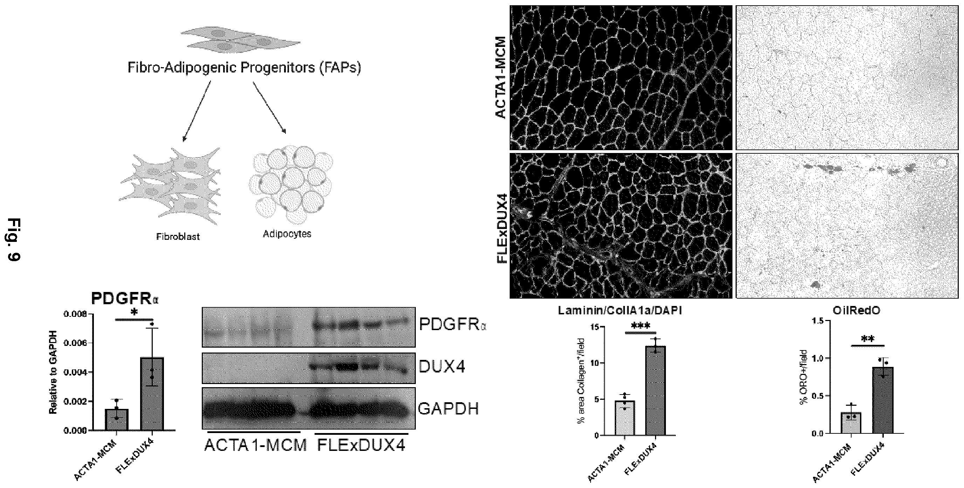

**Figure 9****. Fibro-Adipogenic Progenitor expansion in FLExDUX4 mice.**
   Left. Expansion of FAPs in muscle of FLExDUX4 compared to ACTA1-MCM mice evaluated by quantification of the FAP marker PDGFRalpha at RNA and protein levels.
   Right. Increased fibrosis and fat in muscle of FLExDUX4 compared to ACTA1-MCM mice evaluated by quantification of CollA1a fibrosis marker and OilRedO staining, respectively. N=3. Unpaired t-test, *p<0.05; **p<0.01; ***p<0.001.

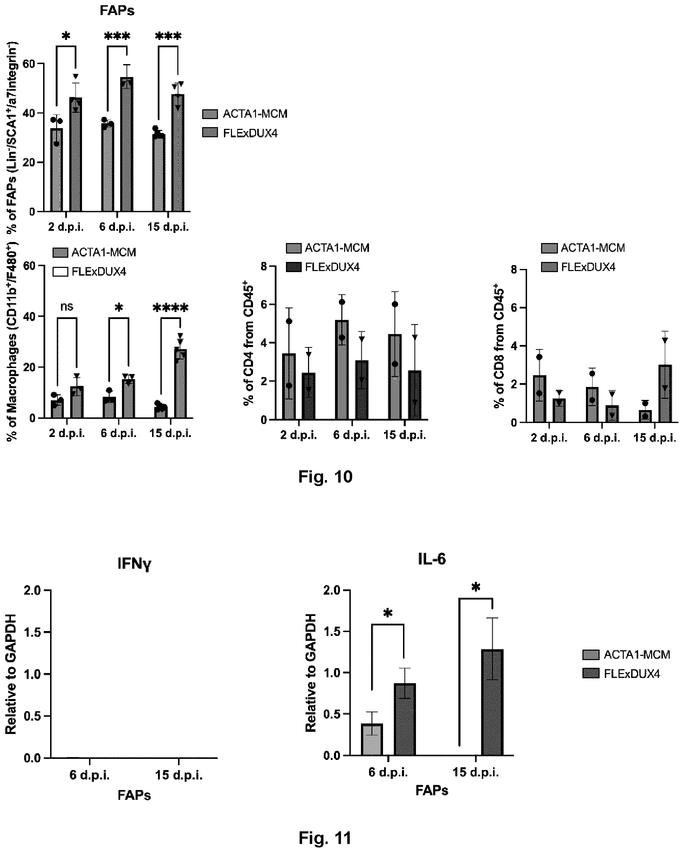

**Figure 10****. FAPs are the first extra-muscular cells affected by DUX4 expression.**
   FAPs, (top), macrophages (bottom left), CD4 (bottom center) and CD8 (bottom right) lymphocytes were isolated by FACS using specific markers and quantified in FLExDUX4 and control muscle at 2, 6 and 15 days post tamoxifen injection (d.p.i.).
   N=2-5. Two-way analysis of variance (ANOVA), with Šídák's and Tukey's multiple comparisons test, *p<0.05; **p<0.01; ***p<0.001.
**Figure 11****. IL-6 but not IFN-γ induction in FLExDUX4 FAPs.**
   IFN-γ and IL-6 RNA levels evaluated by RT-qPCR in FAPs purified from muscle of ACTA1-MCM and FLExDUX4 mice at the indicated days post injection (d.p.i.) of tamoxifen. N=3. Two-way analysis of variance (ANOVA), with Tukey's multiple comparisons test, *p<0.05.

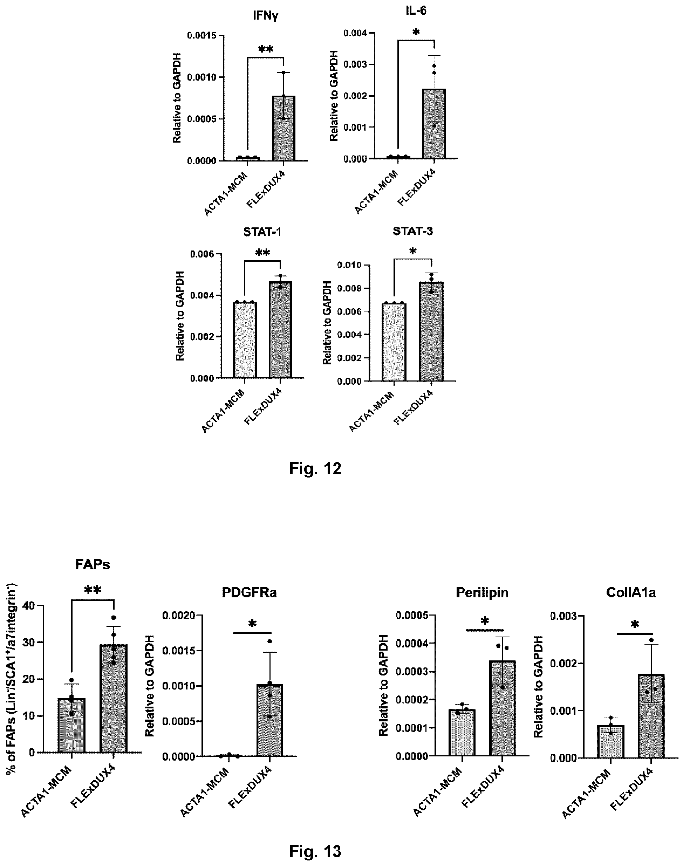

**Figure 12****. Inflammatory factors are activated in the chronic model of FSHD.**
   IFN-γ, IL-6, STAT-1 and STAT-3 RNA levels evaluated by RT-qPCR in muscle from 23 weeks old ACTA1-MCM and FLExDUX4 mice.
   N=3. Unpaired t-test, *p<0.05; **p<0.01.
**Figure 13****. Fibro-Adipogenic Progenitor expansion in the chronic model of FSHD.**
   Left. Expansion of FAPs in muscle of 23 weeks old FLExDUX4 compared to ACTA1-MCM mice evaluated by FACS analysis and quantification of the FAP marker PDGFR alpha. Right. Increased fibrosis and fat in muscle of 23 weeks old FLExDUX4 compared to ACTA1-MCM mice evaluated by quantification of Perilipin fat marker and CollA1a fibrosis marker, respectively.
   N=3-5. Unpaired t-test, *p<0.05; **p<0.01.

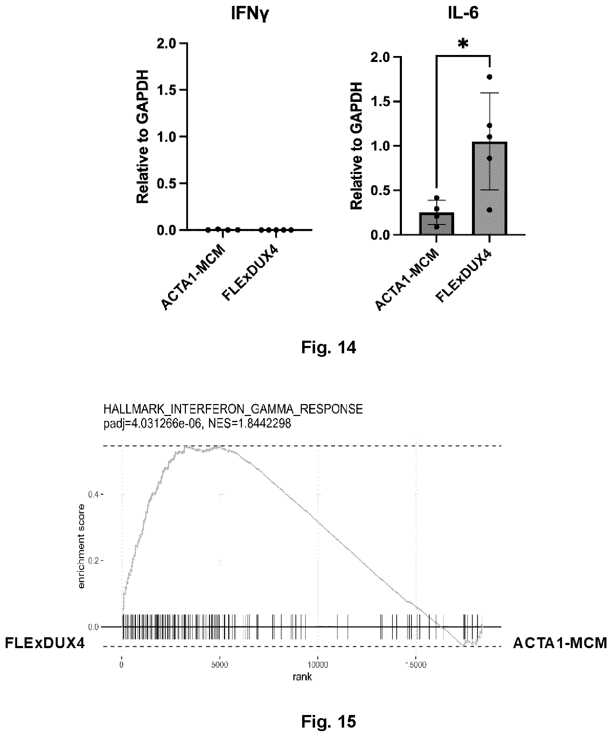

**Figure 14****. IL-6 but not IFN-γ induction in FAPs purified from the chronic model of FSHD.**
   IFN-γ and IL-6 RNA levels evaluated by RT-qPCR in FAPs purified from muscle of 23 weeks old ACTA1-MCM and FLExDUX4 mice.
   N=4-5. Unpaired t-test, *p<0.05.
**Figure 15****. Activation of the IFN-γ response signature in FLExDUX4 mice.**
   Publicly available gene expression datasets using muscle biopsies from control and FLExDUX4 mice treated with tamoxifen used to perform gene set enrichment analysis on the list of genes significantly upregulated in FLExDUX4 compared to control mice.

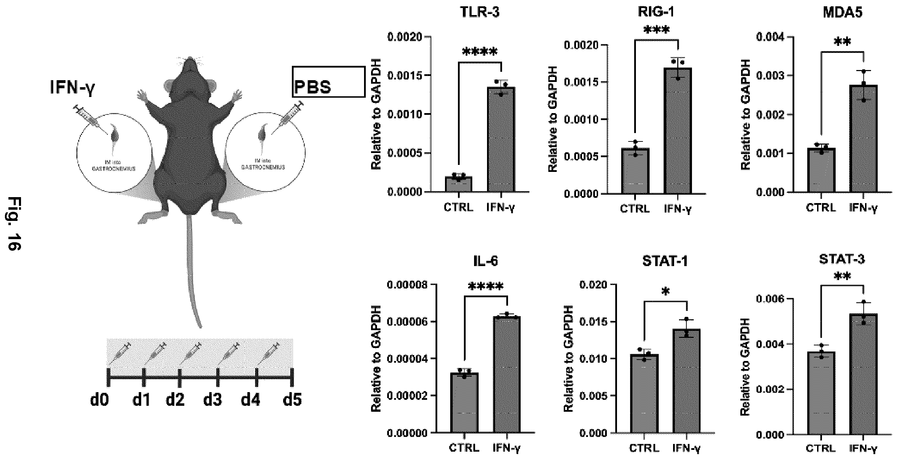

**Figure 16****. IFN-γ is sufficient to induce the viral mimicry inflammatory pathway in WT mice.**
   LEFT. Schematic representation of the IFN-γ treatment in WT mice.
   RIGHT. RNA sensors, IL-6, STAT-1 and STAT-3 RNA levels evaluated by RT-qPCR in PBS (control) and IFN-γ treated mice.
   N=3. Unpaired t-test *p<0.05; **p<0.01: ***p<0.001; ****p<0.0001.

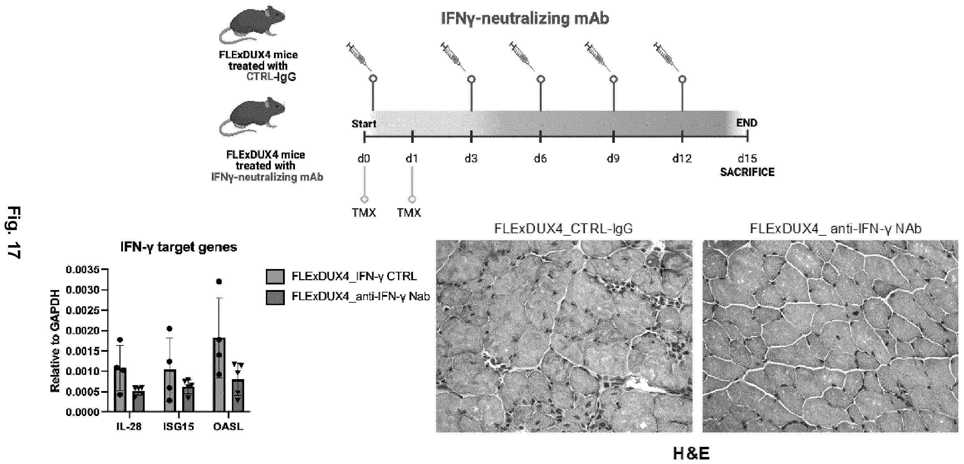

**Figure 17****. Amelioration of FLExDUX4 muscle histology by IFN-γ inhibition.**
   Top. Schematic representation of the anti-IFN-γ neutralizing mAb treatment experimental design.
   Bottom left. RNA levels of genes induced by IFN-γ evaluated by RT-qPCR in control or anti-IFN-γ neutralizing antibodies treated FLExDUX4 mice muscle.
   Bottom right. Histological analysis of control or anti-IFN-γ neutralizing antibodies treated FLExDUX4 mice muscle.

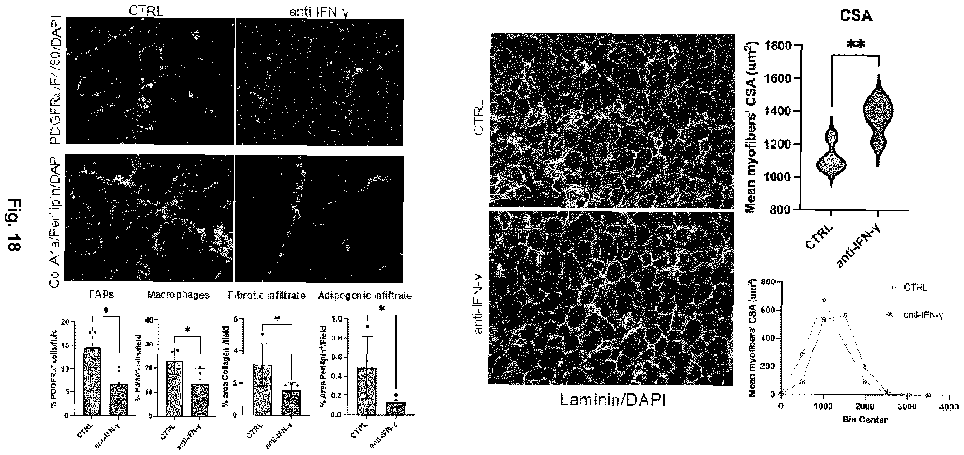

**Figure 18****. IFN-γ inhibition reduces muscle inflammation, FAPs expansion, fibro-fatty conversion, preserving muscle tissue of FLExDUX4 mice.**
   Left. Representative images and quantification of FAPs, macrophages and fibro-fatty substitution in muscle of FLExDUX4 treated with control or anti-IFN-γ neutralizing antibodies.
   Right. Representative images and quantification of muscle fiber cross-sectional area in muscle of FLExDUX4 treated with control or anti-IFN-γ neutralizing antibodies.

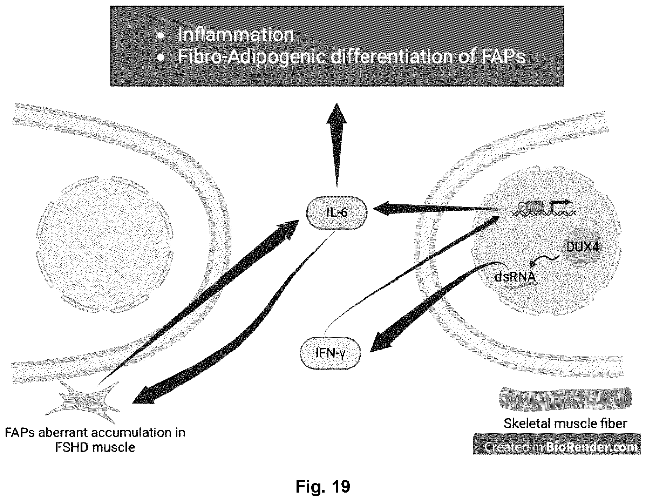

**Figure 19****. Proposed model of DUX4-induced inflammation and muscle wasting.** Aberrant DUX4 expression activates dsRNA production leading to activation of IFN-γ in muscle fibers, which activates STATs. This activates an initial IL-6 secretion from muscle fibers that stimulate FAPs to produce further IL-6, instigating a vicious cycle leading to muscle inflammation, muscle wasting and fat replacement.

### DETAILED DESCRIPTION OF THE INVENTION

IFN-γ, STAT-1, STAT-3 and IL-6 are significantly upregulated in muscle cells of FSHD patients and in muscle tissue of FSHD mouse models. Moreover the activation of IFN-γ is associated with the expansion of Fibro-Adipogenic Progenitors (FAPs) and their conversion to a pro-fibrotic state. Based on these findings and the existing literature, the invention demonstrates that aberrant expression of DUX4 activates a viral mimicry response, resulting in an initial wave of IFN-γ and IL-6 production by skeletal muscle fibers. This, in turn, stimulates FAPs (fibro-adipogenic progenitors) to further IL-6 production and STAT-3 activation, initiating a vicious cycle that leads to muscle inflammation, muscle loss and fat replacement. Elucidating the molecular mechanism through which DUX4 causes FSHD, the invention provides a new strategy for improving the pathophysiology of FSHD.

Therefore the present invention provides an inhibitor of the IFN-γ inflammatory pathway for use in the treatment of FSHD.

As used herein, an "inhibitor of the IFN-γ inflammatory pathway" can target IFN-γ as well as its downstream signaling components, collectively also referred as the IFN-y/IL-6/STAT/JAK inflammatory pathway.

The present invention refers to:
- an inhibitor of IFN-γ expression and/or function; and/or
- an inhibitor of IL-6 expression and/or function; and/or
- an inhibitor of STAT-3 expression and/or function; and/or
- an inhibitor of JAK kinases expression and/or function;
or combination thereof for use in the treatment and/or prevention of FSHD.

Preferably the invention refers to an inhibitor of IFN-γ expression and/or function. Said inhibitor can be used in combination with an inhibitor of IL-6 expression and/or function and/or an inhibitor of STAT-3 expression and/or function.

The inhibitor of the IFN-γ expression and/or function can be used also in combination with an inhibitor of JAK kinases expression and/or function. JAK1 and JAK2 are essential for signal transduction downstream of IFN-γ and IL-6. Consequently, inhibition of JAK cooperates with inhibition of IFN-γ and/or IL-6. Preferably the invention refers to an inhibitor of IFN-γ expression and/or function in combination with an inhibitor of IL-6 expression and/or function and, optionally, an inhibitor of JAK kinases expression and/or function. The invention further refers to an inhibitor of IFN-γ expression and/or function in combination with an inhibitor of STAT-3 expression and/or function and, optionally, an inhibitor of JAK kinases expression and/or function.

The invention demonstrates that DUX4 induces the viral mimicry pathway, leading to activation of an IFN-y/IL-6/STAT inflammatory response in FSHD.

The objects of the present invention may therefore be used to treat and/or prevent any disease or pathology caused by or resulting from the aberrant expression of DUX4, such as FSHD. The disease or pathology caused by or resulting from aberrant expression and/or function of DUX4 is also selected from the group consisting of: muscular dystrophy or cancer. Preferably the cancer is selected from the group consisting of: acute lymphoblastic leukemia, rhabdomyosarcoma, breast, testis, kidney, stomach, lung, thymus, liver, uterus, larynx, esophagus, tongue, heart, connective, mouth, colon, mesothelioma, bladder, ovary, brain, tonsil, pancreas, peritoneum, prostatic or thyroid cancer (Cell Stem Cell. 2018 Dec 6;23(6):794-805.e4 incorporated by reference). Preferably the muscular dystrophy is FSHD. As used herein an inhibitor of IFN-γ or IL-6 or STAT-3 expression and/or function refers to any molecule or agent which reduces the expression of IFN-γ or IL-6 or STAT-3 gene respectively or reduces the activity of IFN-γ or IL-6 or STAT-3 respectively, i.e. the downstream signaling of IFN-γ or IL-6 or STAT-3, respectively. Said inhibitor can be e.g. a nucleic acid, a small molecule, an antibody or any agent which inhibits the expression of IFN-γ or IL-6 or STAT-3.

As used herein an inhibitor of IFN-γ expression and/or function includes IFN-γ neutralizing antibodies, IFN-γ Receptor inhibitors, IFN-γ receptor decoys, agents that block signaling downstream of IFN-γ (like for example the Janus kinase (JAK) 1/2 inhibitors ritlecitinib, Tofacitinib, Filgotinib, Upadacitinib or Abrocitinib), IFN-γ Antagonist 1, IFN-γ antisense oligonucleotides, IFN-γ immunomodulatory agents (like for example GIT 27, Mesopram, or Rocaglamide).

In a preferred embodiment, the anti-IFN-γ antibody is selected from Emapalumab (Gamifant; CAS no. 1709815-23-5; DB14724), fontolizumab (Huzaf; CAS no. 326859-36-3; DB05111), AMG 811 (described in U.S. Pat. No. 7,335,743), AB_2687717 (Bio X Cell Cat# BE0235). More preferably the anti-IFN-γ antibody is from Emapalumab (Gamifant; CAS no. 1709815-23-5; DB14724).

As used herein an inhibitor of IL-6 expression and/or function includes IL-6 Receptor inhibitors, soluble forms of the gp130 receptor (sgp130), IL-6 antisense oligonucleotides, agents that block signaling downstream of IL-6 (like for example the Janus kinase (JAK) 1/2 inhibitors Ruxolitinib, Tofacitinib, Filgotinib, Upadacitinib or Abrocitinib).

In a preferred embodiment the inhibitor of IL-6 expression and/or function is anti-IL-6 antibody, preferably said anti IL-6 antibody is selected from Tocilizumab (CAS no. 375823-41-9; DB06273; commercial names Actemra and RoActemra), Siltuximab (CAS No. 541502-14-1; DB09036; commercial name Sylvant), Sarilumab (CAS No. 1189541-98-7; DB 11767; commercial name Kevzara), ziltivekimab (CAS no. 2226654-05-1), olokizumab (CDP6038; CAS no. 1007223-17-7), elsilimomab (CAS no. 468715-71-1), clazakizumab (BMS-945429, ALD518; CAS no. 1236278-28-6), sirukumab (CNTO 136; CAS no. 1194585-53-9; DB11803), levilimab (BCD-089; CAS no. 2035008-70-7).

As used herein an inhibitor of STAT-3 expression and/or function includes selective STAT3 inhibitors like for example YY002, BBI608 or BP-1-102, STAT3 antisense oligonucleotides, STAT3 decoy oligonucleotides.

Preferably the inhibitor of STAT-3 expression and/or function is a small molecule selected from atovaquone (CAS no. 95233-18-4), WP1066 (CAS no. 857064-38-1), TTI-101 (CAS No. 432001-19-9), VVD-130850, OPB-51602, silibinin (Cas No. 22888-70-6 ) and KT-333. Still preferably the inhibitor of STAT3 expression and/or function is an antisense oligonucleotide inhibitor such as Danvatirsen (AZD9150)

As used herein an inhibitor of JAK kinases expression and/or function refers to any variety of compounds, (small molecules, biological molecules, anti-JAK antibodies, interfering RNA, shRNA, nucleic acids/vectors encoding proteins, CRISPR-Cas mediated systems, etc.) that, when in contact with a Janus kinase enzyme (JAK1, JAK2, JAK3, TYK2) specifically bind with a Janus kinase enzyme altering the JAK-STAT signaling pathway. Preferably, an inhibitor of JAK kinases, as herein used, refers to an inhibitor of JAK1 and/or JK2. Examples include baricitinib (Olumiant), tofacitinib (Xeljanz), ruxolitinib (Jakavi, Opzelura), abrocitinib (Cibinqo), upadacitinib (Rinvoq), fedratinib (Inrebic), ritlecitinib (Litfulo), cerdulatinib, gandotinib, lestaurtinib, momelotinib, pacritinib, oclacitinib, peficitinib, filgotinib, delgocitinib, all of which are contemplated for uses disclosed herein. The term "molecule" in the present invention includes the term "inhibitor of ..." as defined herein.

In the present invention, the herein defined molecules or inhibitors may be used in combinations.

In a preferred embodiment of the invention, IFN-γ is a protein and it preferably has essentially the amino acid sequences disclosed as NCBI Accession no. NP_000610.2, AAA53230, AAM28885, AAB59534, AAH70256, BAG70267, BAG70138, AAP20098, CAA23804.

In another preferred embodiment, IFN-γ is a gene and it preferably has essentially the nucleotide sequence disclosed as NCBI Accession no. NG_015840.1.

In another preferred embodiment, IFN-γ is a mRNA and it preferably has essentially the nucleotide sequences disclosed as NCBI Accession no. NM_000619.3, X13274, X01992, V00543, AY255837, AB451324, AB451453, BC070256, CAA26022, CAA31639.

In preferred embodiment, IL-6 is a protein and it preferably has essentially the amino acid sequences disclosed as NCBI Accession no. NP_000591.1, NP_001305024.1, NP_001358025.1, XP_005249802.1, XP_054214121.1, XP_054214120.1.

In another preferred embodiment, IL-6 is a gene and it preferably has essentially the nucleotide sequence disclosed as NCBI Accession no. NG_011640.1

In another preferred embodiment, IL-6 is a mRNA and it preferably has essentially the nucleotide sequences disclosed as NCBI Accession no. NM_000600.5, NM_001318095.2, NM_001371096.1, XM_005249745.6, XM_054358146.1, XM_054358145.1.

In preferred embodiment, STAT-3 is a protein and it preferably has essentially the amino acids sequences disclosed as NCBI Accession no. NP_001356441.1, NP_001356442.1, NP_001356443.1, NP_001356445.1, NP_001356446.1, NP_001356447.1, NP_001356448.1, NP_001371918.1, NP_001371921.1, NP_001371913.1, NP_003141.2, NP_001371914.1, NP_001371916.1, NP_001371920.1, NP_001371917.1, NP_001356449.1, NP_998827.1, NP_001371922.1, NP_644805.1, NP_001371915.1, NP_001371919.1, XP_047292542.1, XP_016880462.1, XP_047292541.1, XP_054172968.1, XP_054172967.1, XP_054172966.1. In another preferred embodiment, STAT-3 is a gene and it preferably has essentially the nucleotide sequence disclosed as NCBI Accession no. NG_007370.1.

In another preferred embodiment, STAT-3 is a mRNA and it preferably has essentially the nucleotide and amino acids sequences disclosed as NCBI Accession no. NM_001384989.1, NM_001384992.1, NM_001384984.1, NM_001369519.1, NM_003150.4, NM_001369517.1, NM_001369512.1, NM_001369514.1, NM_001369518.1, NM_001369513.1, NM_001384985.1, NM_001384987.1, NM_001384991.1, NM_001384988.1, NM_001369520.1, NM_001369516.1, NM_213662.2, NM_001384993.1, NM_139276.3, NM_001384986.1, NM_001384990.1, XM_047436586.1, XM_017024973.3, XM_047436585.1, XM_054316993.1, XM_054316992.1

In a preferred embodiment JAK 1 is a protein and it preferably has essentially the amino acids sequences disclosed as NCBI Accession no. XP_047275632.1, NP_001308781.1, NP_001308783.1, NP_001308782.1, NP_001308785.1, NP_001307852.1, NP_001308786.1, NP_002218.2, NP_001308784.1, XP_047275633.1, XP_047275631.1, XP_047275630.1, XP_054192402.1, XP_054192403.1, XP_054192401.1.

In another preferred embodiment, JAK 1 is a gene and it preferably has essentially the nucleotide sequence disclosed as NCBI Accession no. 3716.

In another preferred embodiment, JAK 1 is a mRNA and it preferably has essentially the nucleotide and amino acids sequences disclosed as NCBI Accession no. XM_047419676.1, NM_001321852.2, NM_001321854.2, NM_001321853.2, NM_001321856.2, NM_001320923.2, NM_001321857.2, NM_002227.4, NM_001321855.2, XM_047419677.1, XM_047419675.1, XM_047419674.1, XM_054336427.1, XM_054336428.1, XM_054336426.1

In a preferred embodiment JAK 3 is a protein and it preferably has essentially the amino acids sequences disclosed as NCBI Accession no. NP_000206.2, XP_047294742.1, XP_011526293.2, XP_054176885.1, XP_054176886.1.

In another preferred embodiment, JAK 3 is a gene and it preferably has essentially the nucleotide sequence disclosed as NCBI Accession no. 3718.

In another preferred embodiment, JAK 3 is a mRNA and it preferably has essentially the nucleotide and amino acids sequences disclosed as NCBI Accession no. XR_007066796.1, NM_000215.4, XM_047438786.1, XM_011527991.3, XR_008485140.1, XM_054320910.1, XM_054320911.1.

In a preferred aspect of the invention, the inhibitor is an antibody or synthetic or recombinant derivative thereof.

Said antibody is preferably a monoclonal or polyclonal antibody, or synthetic or recombinant derivatives thereof, more preferably said antibody being a humanized monoclonal antibody. Preferably, said polynucleotide is a RNA or DNA.

Preferably said polynucleotide able to inhibit the expression of the IFN-γ or IL-6 or STAT-3 or JAK kinases is an RNA inhibitor, more preferably it is a siRNA, a shRNA, a microRNA or an antisense oligonucleotide.

In a preferred embodiment, the invention refers to an expression vector comprising or expressing the polynucleotide as defined above, and said vector is selected from the group consisting of: plasmids, viral particles and phages.

In a further embodiment, the invention refers to a host cell comprising the above vector and which is selected from the group consisting of: bacterial cells, fungal cells, insect cells, animal cells, plant cells, preferably being an animal cell, more preferably a human cell.

By the term "inhibitor" it is meant a molecule that effects a change in the expression and/or function of the target. The change is relative to the normal or baseline level of expression and/or function in the absence of the molecule, but otherwise under similar conditions, and it represent a decrease in the normal/baseline expression and/or function.

The terms "function" and "activity" are herein interchangeable.

The inhibition (which includes blocking) of the expression and/or function of the target may be assessed by any means known to the skilled in the art. The assessment of the expression level or of the presence of the target is preferably performed using classical molecular biology techniques such as real time Polymerase Chain Reaction (qPCR), microarrays, bead arrays, RNAse protection analysis or Northern blot analysis or cloning and sequencing, or next generation sequencing.

The assessment of target function (or activity) is e.g. performed by analysis of targets genes, chromatin association, chromatin modifications, chromatin accessibility, cell adhesion, cell migration, cell proliferation, cell death.

In the context of the present invention, the target may be the gene, the pre-mRNA, the mRNA, the cDNA, or the encoded protein thereof.

The polynucleotides as above described, as e.g. the siRNAs, may further comprise dTdT or UU 3'-overhangs, and/or nucleotide and/or polynucleotide backbone modifications as described elsewhere herein.

The invention also refers to an in vitro method for evaluating the risk and / or for diagnosis and / or for prognosis and/or for monitoring progression and / or for monitoring the efficacy of a therapeutic treatment and/or for screening of FSHD in a subject or in an ex vivo or in vitro system, preferably an organoid such as artificial skeletal muscle.

In the method of the invention, preferably step a) comprises measuring the amount of the protein IFN-γ or IL-6 or STAT-3 or of fragments thereof or of the polynucleotide coding for said proteins or of fragments thereof in said isolated biological sample obtained from the subject and step b) comprises comparing the measured amount of step a) with a proper control amount.

Preferably, the in vitro method as above defined preferably comprises the steps of:
a) measuring the amount or the activity of the protein IFN-γ or IL-6 or STAT-3 or of fragments thereof or of the polynucleotide coding for said protein or fragments thereof in said isolated biological sample obtained from the subject and
b) comparing the measured amount or activity of step a) with a proper control amount or activity, wherein an amount or activity of said protein IFN-γ or IL-6 or STAT-3 or of fragments thereof or of said polynucleotide or of fragments thereof in the isolated biological sample obtained from the subject higher than the control amount or activity indicates that the subject is either at increased risk for developing or is affected by the pathologies herein mentioned.

Preferably, the in vitro method for monitoring the progression and/or for monitoring the efficacy of a therapeutic treatment of one of the pathologies herein mentioned comprises the steps of:
a) measuring the alteration of the amount or the alteration of the activity of the IFN-γ or IL-6 or STAT-3 proteins or of fragments thereof or of the polynucleotide coding for said protein or fragments thereof in said isolated biological sample obtained from the subject and
b) comparing the measured alteration of step a) with a proper control alteration.

Preferably, the biological sample is a fluid, a cell or a tissue sample, more preferably said sample is plasma or serum or blood.

Another object of the invention is a kit for carrying out the above methods, comprising
- means to measure the amount or the activity of the IFN-γ or IL-6 or STAT-3 proteins or of fragments thereof and/or means to measure the amount of the polynucleotide coding for said protein or of fragments thereof and optionally,
- control means.

Control means can be used to compare the amount or the increase of amount of the compound as above defined to a proper control. The proper control may be obtained for example, with reference to known standard, either from a normal subject or from normal population. The means to measure the amount of at least one compound as above defined are preferably at least one antibody, functional analogous or derivatives thereof. Said antibody, functional analogous or derivatives thereof are specific for said compound.

In a preferred embodiment, the kit of the invention comprises:
- a solid phase adhered antibody specific for said compound;
- detection means of the ligand specific-biomarker complex. Other means may be e.g. specific primers and probes for RT-PCR.

The kits according to the invention can further comprise customary auxiliaries, such as buffers, carriers, markers, etc. and/or instructions for use.

In the case of a method or a kit for assessing the risk and/or diagnosing and/or prognosing of a disease herein mentioned, the proper control may be a sample taken from a healthy patient or from a patient affected by another disorder or pathology not characterized by DUX4 aberrant expression, and the proper control amount or activity may be the amount or activity of the same protein or polynucleotide measured in a sample taken from a healthy patient or from a patient affected by another disorder or pathology not characterized by DUX4 aberrant expression.

In the case of a method or a kit for monitoring the progression of the disease, the progress of the disease is monitored and the proper control may be a sample taken from the same subject at various times or from another patient, and the proper control amount or activity may by the amount or activity of the same protein or polynucleotide measured in a sample taken from the same subject at various times or from another patient.

In the case of a method or a kit for monitoring the efficacy of a therapeutic treatment, the proper control may by a sample taken from the same subject before initiation of the therapy or taken at various times during the course of the therapy and the proper control amount or activity may be the amount or activity of the same protein or polynucleotide measured in a sample taken from the same subj ect before initiation of the therapy or taken at various times during the course of the therapy. In the case of a method or a kit for the screening of a therapeutic treatment, the proper control may be a sample taken from subjects without treatment and from subjects treated with a substance that is to be assayed or from subjects treated with a reference treatment and the proper control amount or activity may be the average of the amounts or activity of the same protein or polynucleotide measured in samples taken from subjects without treatment and from subjects treated with a substance that is to be assayed or from subjects treated with a reference treatment. In this case, if the amount or activity of said protein or of said polynucleotide in the isolated biological sample obtained from the subject is lower or equal than the control amount or activity, it may indicate that the tested substance is effective for the treatment of the herein mentioned disease.

In the present invention, the expression "measuring the amount" can be intended as measuring the amount (or the activity) or concentration or level of the respective protein and/or mRNA thereof and/or DNA thereof, preferably semi-quantitative or quantitative. Measurement of a protein can be performed directly or indirectly. Direct measurement refers to the amount or concentration measure of the biomarker, based on a signal obtained directly from the protein, and which is directly correlated with the number of protein molecules present in the sample. This signal - which can also be referred to as intensity signal - can be obtained, for example, by measuring an intensity value of a chemical or physical property of the biomarker. Indirect measurements include the measurement obtained from a secondary component (e.g., a different component from the gene expression product) and a biological measurement system (e.g. the measurement of cellular responses, ligands, "tags" or enzymatic reaction products).

The term "amount", as used in the description refers but is not limited to the absolute or relative amount of proteins and/or mRNA thereof and/or DNA thereof, and any other value or parameter associated with the same or which may result from these. Such values or parameters comprise intensity values of the signal obtained from either physical or chemical properties of the protein, obtained by direct measurement, for example, intensity values in an immunoassay, mass spectroscopy or a nuclear magnetic resonance. Additionally, these values or parameters include those obtained by indirect measurement, for example, any of the measurement systems described herein. Methods of measuring mRNA and DNA in samples are known in the art. To measure nucleic acid levels, the cells in a test sample can be lysed, and the levels of mRNA in the lysates or in RNA purified or semi-purified from lysates can be measured by any variety of methods familiar to those in the art. Such methods include hybridization assays using detectably labeled DNA or RNA probes (i.e., Northern blotting) or quantitative or semi-quantitative RT-PCR methodologies using appropriate oligonucleotide primers. Alternatively, quantitative or semi-quantitative in situ hybridization assays can be carried out using, for example, tissue sections, or unlyzed cell suspensions, and detectably labeled (e.g., fluorescent, or enzyme-labeled) DNA or RNA probes. Additional methods for quantifying mRNA include RNA protection assay (RPA), cDNA and oligonucleotide microarrays, representation difference analysis (RDA), differential display, EST sequence analysis, and serial analysis of gene expression (SAGE) and next generation sequencing (RNA-sequencing).

If by comparing the measured amount or activity of the protein or of the polynucleotide coding for said protein with the amount or activity obtained from a control sample, the amount or the activity of said compound in the sample isolated from the subject corresponds to a higher value, the subject may present the disease or go towards an aggravation of said disease.

If by comparing the measured amount or activity of the protein or of the polynucleotide coding for said protein with the amount or the activity obtained from a control sample, the amount or the activity of said compound in the sample isolated from the subject corresponds to a similar or lower value, the subject may be not affected by the disease or go toward an amelioration of the disease, respectively.

Alternatively, the expression "detection" or "measuring the amount" is intended as measuring the alteration of the molecule. Said alteration can reflect an increase or a decrease in the amount or activity of the molecules as above defined. An increase of the protein or of the activity of the protein or of the polynucleotide coding for said protein can be correlated to an aggravation of the disease.

A decrease of the protein or of the activity of the protein or of the polynucleotide coding for said protein can be correlated to an amelioration of the disease or to recovery of the subject. The term "biological sample" encompasses a clinical sample, and also includes tissue obtained by surgical resection, tissue obtained by biopsy, cells in culture, cell supernatants, cell lysates, tissue samples, organs, blood, plasma, serum, and the like.

A "sample" in the context of the present teachings refers to any biological sample that is isolated from a subject. A sample can include, without limitation an aliquot of body fluid, whole blood, serum, plasma, solid tissue samples such as tissue biopsies, or tissue cultures or cells derived therefrom and the progeny thereof, synovial fluid, lymphatic fluid, ascites fluid, and interstitial or extracellular fluid. The term "sample" also encompasses the fluid in spaces between cells, including gingival crevicular fluid, bone marrow, cerebrospinal fluid (CSF), saliva, mucous, sputum, semen, sweat, urine, or any other bodily fluids. "Blood sample" can refer to whole blood or any fraction thereof, including serum and plasma. Samples can be obtained from a subject by means including but not limited to venipuncture, excretion, ejaculation, massage, biopsy, needle aspirate, lavage, scraping, surgical incision, or intervention or other means known in the art.

In the methods of the invention, the subject (or patient) may be an animal, preferably a mammalian, or a human.

Further assessment methods include in vitro suppression assay, whole transcriptome analysis, mass spectrometry analysis to identify proteins interacting with the target.

The polynucleotide according to the invention includes also chemically and structurally modified polynucleotide, preferably siRNAs, to solve their intrinsic problems related to in vivo instability, off-target effects, and immunogenicity.

The vector according to the invention also includes novel vector-driven bifunctional short hairpin RNA (bi-shRNA) (Ku SH., et al. 2015; Mohr SE,et al. 2014).

The vector of the invention also includes an RNAi-inducing vector whose presence within a cell results in production of an siRNA or shRNA or miRNA, preferably siRNA.

One or more siRNAs or shRNAs (in any combination) may be used.

Such polynucleotides can be single or double stranded. Preferably, one strand of a double-stranded polynucleotide comprises at least a partial sequence complementary to a target mRNA. The nucleotides of the inhibitory nucleic acid can be chemically modified, natural or artificial. The sequence homology between the polynucleotide (e.g. a RNAi inducing agent) and the target mRNA may be 100% or less, but is ideally greater than about 50% and typically 90% or greater and even more preferably at least 98% and 99%. It will be understood that the percentage of sequence homology between RNAi inducing agent and the target mRNA should be sufficient to result in sequence specific association between the RNAi inducing agent, e.g. siRNA, and the target mRNA, preferably under cytoplasmic conditions.

Such siRNAs comprise two RNA strands having a region of complementarity of approximately 20 or so nucleotides in length and optionally further comprises one or two single-stranded overhangs or loops. In mammalian cells, dsRNA longer than 30 base pairs can cause nonspecific gene suppression by an interferon a response. However, cells transfected with 21 nucleotide synthetic double-stranded siRNA bearing two nucleotides protruding at both 3 '-ends have been found to escape an interferon response and effectively exert sequence-specific gene silencing function. The silencing effect of the synthetic siRNA, however, is transient. The double stranded siRNA molecule down regulates expression of the IFN-γ or II,6 or STAT3 protein via RNAi, wherein each strand of said siRNA molecule is independently about 18 to about 28 nucleotides in length and one strand of the siRNA molecule comprises a nucleotide sequence having sufficient complementarity to the RNA of the target protein for the siRNA molecule to direct cleavage of the target RNA via RNA interference. In shRNA, the single RNA strand may form a hairpin structure with a stem and loop and, optionally, one or more unpaired portions at the 5' and/or 3' portion of the RNA.

The delivery of an effective amount of the polynucleotide according to the invention, may be carried out with any know method which is suitable for the delivery. For example, some delivery agents for the siRNA, microRNA, etc. are selected from the following non-limiting group of cationic polymers, modified cationic polymers, peptide molecular transporters, lipids, liposomes and/or non-cationic polymers. Viral vector delivery systems may also be used. For example, an alternative delivery route includes the direct delivery of the polynucleotide (including siRNA, shRNA and miRNA) and even anti-sense RNA (asRNA) in gene constructs followed by the transformation of cells with the resulting recombinant DNA molecules. This results in the transcription of the gene constructs encoding the polynucleotide, such as siRNA, shRNA and miRNA, or even asRNA and provides for the transient and stable expression of the polynucleotide in cells and organisms. For example, such an alternative delivery route may involve the use of a lentiviral vector comprising a nucleotide sequence encoding a shRNA which targets the IFN-γ or IL6 or STAT3. Such a lentiviral vector may be comprised within a viral particle. Adeno-associated viruses (AAV) may also be used and the use of these as delivery vehicles is expanded on later. siRNA delivery formulations include e.g. siRNA conjugates, polymerized siRNA, and nucleic acid-based nanoparticles to improve in vivo delivery.

RNA interference (RNAi) is the process by which expression of a target gene is effectively silenced or knocked down by the selective inactivation of its corresponding mRNA by double-stranded RNA (dsRNA). RNAi is activated by dsRNA species delivered to the cytoplasm of cells. The silencing mechanisms can either lead to the degradation of a target mRNA, as induced by small interfering RNAs (siRNAs) or short hairpin RNAs (shRNAs), or the suppression of translation of specific mRNAs, as induced by microRNA (miRNA). Two key approaches to RNAi that have gained substantial interest for use in gene silencing are the double-stranded small interfering RNAs (siRNAs) and the vector-based short hairpin RNAs (shRNAs).

Both these methods can achieve robust and specific knockdown, but they have striking mechanistic differences with broad practical implications. shRNA can effectively target knockdown with low copy numbers and longer lasting effects than siRNA. The recently introduced bifunctional design has similar benefits to standard shRNA but with greatly enhanced potency.

The use of shRNA is suggested by the advantages of shRNA over siRNA including the ability to use viral vectors for delivery to overcome the difficulty of transfecting certain cell types, the option to control shRNA expression using inducible promoters, and the ability to co-express them with a reporter gene. Additionally, they may cause fewer off-target effects. Furthermore, novel vector-driven bifunctional short hairpin RNA (bi-shRNA) technology that harnesses both cleavage-dependent and cleavage-independent RISC loading pathways to enhance knockdown potency may be used. Indeed, consequent advantages provided by the bi-shRNA include a lower effective systemic dose than comparator siRNA/shRNA to minimize the potential for off-target side effects, due to its ability to induce both a rapid (inhibition of protein translation) and delayed (mRNA cleavage and degradation) targeting effect depending on protein and mRNA kinetics, and a longer duration of effectiveness for clinical applications. The RNAi inhibitors as above defined are preferably capable of hybridizing to all or part of specific target sequence of IFN-γ or IL6 or STAT3. Therefore, RNAi inhibitors may be fully or partly complementary to all of or part of the target sequence.

The RNAi inhibitors may hybridize to the specified target sequence under conditions of medium to high stringency.

RNAi inhibitors may be defined with reference to a specific sequence identity to the reverse complement of the sequence to which it is intended to target. The antisense sequences will typically have at least about 75%, preferably at least about 80%, at least about 85%, at least about 90%, at least about 95% or at least about 99% sequence identity with the reverse complements of then-target sequences. The antisense nucleic acid may be Gapmers.

A "derivative" may be a nucleic acid molecule, as a DNA molecule, coding the polynucleotide as above defined, or a nucleic acid molecule comprising the polynucleotide as above defined, or a polynucleotide of complementary sequence. In the context of the present invention the term "derivatives" also refers to longer or shorter polynucleotides and/or polynucleotides having e.g. a percentage of identity of at least 41 %, 50 %, 60 %, 65 %, 70 % or 75%, more preferably of at least 85%, as an example of at least 90%, and even more preferably of at least 95% with the sequences herein disclosed or with their complementary sequence or with their DNA or RNA corresponding sequence. The term "derivatives" and the term "polynucleotide" also include modified synthetic oligonucleotides. The modified synthetic oligonucleotide are preferably LNA (Locked Nucleic Acid), phosphoro-thiolated oligos or methylated oligos, morpholinos, 2'-0-methyl, 2 -0-methoxyethyl oligonucleotides and cholesterol-conjugated 2'-0-methyl modified oligonucleotides (antagomirs).

The term "derivative" may also include nucleotide analogues, i.e. a naturally occurring ribonucleotide or deoxyribonucleotide substituted by a non-naturally occurring nucleotide. The modified nucleotide analog may be located for example at the 5'-end and/or the 3 '-end of the nucleic acid molecule. Nucleotide analogs may be selected from sugar- or backbone-modified ribonucleotides or nucleobase-modified ribonucleotides, i.e. ribonucleotides, containing a non-naturally occurring nucleobase instead of a naturally occurring nucleobase.

The term "derivatives" also includes nucleic acids that may be generated by mutating one or nucleotide in their sequences, equivalents or precursor sequences. The term "derivatives" also includes at least one functional fragment of the polynucleotide.

In the context of the present invention "functional" is intended for example as "maintaining their activity".

As used herein "fragments" refers to polynucleotides having preferably a length of at least 1000 nucleotides, 1100 nucleotide, 1200 nucleotides, 1300 nucleotides, 1400 nucleotides, 1500 nucleotides. Where the fragments are referred to the siRNA as above defined, the length is preferably of 10, 11, 12, 13, 14, 15, 16, 17, 18,19, 20, 21 nucleotides.

The term "polynucleotide" also refers to modified polynucleotides.

According to the present invention, an "effective amount" of a composition is one that is sufficient to achieve a desired biological effect, in this case an amelioration or the treatment of disease herein mentioned.

It is understood that the effective dosage will be dependent upon the age, sex, health, and weight of the recipient, kind of concurrent treatment, if any, frequency of treatment, and the nature of the effect desired. The provided ranges of effective doses of the inhibitor or molecule of the invention (e.g. from 1 mg/kg to 100 mg/kg, in particular systemically administered) are not intended to limit the invention and represent preferred dose ranges. However, the preferred dosage can be tailored to the individual subject, as is understood and determinable by one of skill in the art, without undue experimentation.

The administration of oligonucleotides of the present invention may be carried out by known methods, wherein a nucleic acid is introduced into a desired target cell in vitro or in vivo.

An aspect of the present invention comprises a nucleic acid construct comprised within a delivery vehicle. A delivery vehicle is an entity whereby a nucleotide sequence can be transported from at least one media to another. Delivery vehicles may be generally used for expression of the sequences encoded within the nucleic acid construct and/or for the intracellular delivery of the construct. It is within the scope of the present invention that the delivery vehicle may be a vehicle selected from the group of RNA based vehicles, DNA based vehicles/vectors, lipid-based vehicles, virally based vehicles and cell based vehicles. Examples of such delivery vehicles include: biodegradable polymer microspheres, lipid-based formulations such as liposome carriers, coating the construct onto colloidal gold particles, lipopolysaccharides, polypeptides, polysaccharides, pegylation of viral vehicles.

In one embodiment of the present invention may comprise a virus as a delivery vehicle, where the virus may be selected from: adenoviruses, retroviruses, lentiviruses, adeno-associated viruses, herpesviruses, vaccinia viruses, foamy viruses, cytomegaloviruses, Semliki forest virus, poxviruses, RNA virus vector and DNA virus vector. Such viral vectors are well known in the art.

Commonly used gene transfer techniques include calcium phosphate, DEAE-dextran, transfection, electroporation and microinjection and viral methods. Another technique for the introduction of DNA into cells is the use of cationic liposomes. Commercially available cationic lipid formulations are e.g. Tfx 50 (Promega) or Lipofectamin 3000 (Life Technologies).

The above pharmaceutical compositions are preferably for systemic, oral, locally, preferably rectally, or topical administration.

The compositions of the present invention may be in form of a solution, e.g. an injectable solution, a cream, ointment, tablet, suspension or the like. The composition may be administered in any suitable way, e.g. by injection, particularly by intraocular injection, by oral, topical, nasal, rectal application etc. The carrier may be any suitable pharmaceutical carrier. Preferably, a carrier is used, which is capable of increasing the efficacy of the RNA molecules to enter the target-cells. Suitable examples of such carriers are liposomes, particularly cationic liposomes.

The recombinant expression vector of the invention can be any suitable recombinant expression vector, and can be used to transform or transfect any suitable host. Suitable vectors include those designed for propagation and expansion or for expression or both, such as plasmids and viruses. The recombinant expression vectors of the invention can be prepared using standard recombinant DNA techniques. Constructs of expression vectors, which are circular or linear, can be prepared to contain a replication system functional in a prokaryotic or eukaryotic host cell. Replication systems can be derived, e.g., from CoIEl, 2 µ plasmid, λ, SV40, bovine papilloma virus, and the like.

Desirably, the recombinant expression vector comprises regulatory sequences, such as transcription and translation initiation and termination codons, which are specific to the type of host (e.g., bacterium, fungus, plant, or animal) into which the vector is to be introduced, as appropriate and taking into consideration whether the vector is DNA- or RNA-based. The recombinant expression vector can include one or more marker genes, which allow for selection of transformed or transfected hosts. Marker genes include biocide resistance, e.g., resistance to antibiotics, heavy metals, etc., complementation in an auxotrophic host to provide prototrophy, and the like. Suitable marker genes for the inventive expression vectors include, for instance, neomycin/G418 resistance genes, hygromycin resistance genes, histidinol resistance genes, tetracycline resistance genes, and ampicillin resistance genes. The recombinant expression vector can comprise a native or normative promoter operably linked to the nucleotide sequence encoding the IFN-γ or IL6 or STAT3 inhibitor (including functional portions and functional variants thereof), or to the nucleotide sequence which is complementary to or which hybridizes to the nucleotide sequence encoding the RNA. The selection of promoters, e.g., strong, weak, inducible, tissue- specific and developmental-specific, is within the ordinary skill of the artisan. Similarly, the combining of a nucleotide sequence with a promoter is also within the skill of the artisan. The promoter can be a non-viral promoter or a viral promoter, e.g., a cytomegalovirus (CMV) promoter, an SV40 promoter, an RSV promoter and a promoter found in the long-terminal repeat of the murine stem cell virus.

In the above compositions further materials as well as processing techniques and the like may be set out in Part 5 of Remington's Pharmaceutical Sciences, 20th Edition, 2000, Marck Publishing Company, Easton, Pennsylvania, which is incorporated herein by reference.

The compounds of this invention can also be administered in sustained release forms or from sustained release drug delivery systems. A description of representative sustained release materials can also be found in the incorporated materials in Remington's Pharmaceutical Sciences. Furthermore, pharmaceutical formulations can be prepared using a process, which is generally known in the pharmaceutical art.

In the present invention, when the molecule of the invention is administered with another therapeutic agent, it may be administered simultaneously or sequentially.

In the methods of the invention, the subject (or patient) may be an animal, preferably a mammalian, or a human.

### METHODS

### Animals

B6(Cg)-Gt(ROSA)26Sortm1.1(DUX4*)Plj/J (JAX 028710) and Tg(ACTA1-cre/Esr1*)2Kesr/J (JAX 025750) mice were purchased from The Jackson Laboratory (Maine, USA). FLExDUX4 colony was maintained as homozygous for Gt (ROSA)26Sortm1.1(DUX4*)Plj, while HSA-MCM colony was maintained as hemizygous for Tg (ACTA1-cre/Esr1*)2Kesr. 8/10 weeks-old female mice were used for all the in vivo experiment. Mice were kept and handled in accordance with the animal protocols. All the mouse experiments were approved by the Institutional Animal Care and Use Committee of San Raffaele and by the Italian Ministry of Health (Rome, Italy). The mice that were bred in-house and housed in a temperature and humidity-controlled room in a specified pathogen-free environment under 12:12 h light/dark cycles.

### Transgenic mouse genotyping

DNA was isolated from 2 mm tail snips from <10-day-old mice. The DNA extraction and the PCR were performed whit Thermo Scientific Phire Tissue Direct PCR Master Mix #F-170S following the manufactory instruction and for the PCRs were using the primers reported in the Jackson Laboratory instruction for these particular strains.

### Tamoxifen (TMX) administration

Tamoxifen (TMX) administration was performed as in (2018) PLOS ONE | https://doi.org/10.1371/journal.pone.0192657. Specifically, the intraperitoneal (IP) injections of TMX (Sigma #T5648) was dissolved in 100% ethanol at 55 °C and added to warm sterile corn oil to make a 20mg/ml stock. Stock TMX aliquots were warmed to 37°C, briefly, and diluted further with warm corn oil just prior to use. 8-10 weeks-old mice were weighed and injected IP on two consecutive days (d0 and d1) with the appropriate volume (100µl for a 20g mouse) of TMX for a final concentration of 5 mg/kg.

### Anti-IFN-γ in vivo treatment

8-10 weeks-old female mice were used for the in vivo treatment. Specifically, the bi-transgenic mice ACTA1-MCM/FLExDUX4 (FLExDUX4) mice were injected with TMX (at final concentration of 5 mg/kg) at d0 and d1. Additionally, mice were intraperitoneal (IP) injected with InVivoMAb anti-mouse IFN-γ (Clone H22) neutralizing antibody (BioxCell Catalog #BE0312, RRID AB_2736992) or InVivoMAb polyclonal Armenian hamster IgG (BioxCell Catalog #BE0091, RRID AB_1107773), as control, every three days. Mice were sacrificed at 15 d.p.i. upon tamoxifen treatment.

### IFN-γ in vivo treatment

8-10 weeks-old female mice (ACTA1-MCM) were injected intramuscularly (IM) with IFN-γ 7500U (R&D Biotechne 485-MI) for five consequently days. The contralateral leg was injected via IM with PBS 1X as control.

### Human-IFN-y in vitro treatment

Human primary muscle cells from healthy donors were treated with IFN-γ (R&D Biotechne 285-IF) 25ng/ml for 48h. IFN-γ was added to the differentiation media at day 2 and the cells was harvested at day 4 of differentiation.

### Cell preparation and fluorescence-activated cell sorting

Cell isolation and labeling were essentially performed as described in Biferali et al., Sci. Adv. 2021; 7: eabd9371. Specifically, the single cells suspension was stained with Aqua (1:100 in PBS 1x) for 10 min at 4°C to identify the live cells; then, after one wash in HBSS+ 0,2% BSA+ 1%P/S, the single cells suspension was stained with CD31/CD45/TER119-Biotin (Miltenyi Biotec; 1:25) for lineage exclusion and FAPs were isolated as α7integrin-APC (Miltenyi Biotec; 1:25) negative and Sca1-FITC (Miltenyi Biotec; 1:25) positive cells, meanwhile Macrophages were isolated starting from the CD31/CD45/TER119-Biotin positive cells as F4/80 Pe Vio770 (Miltenyi Biotec; 1:50) and Cd11b APC Vio770 (Miltenyi Biotec; 1:50) double positive cells. Indeed, to identify the CD4 and CD8 population the single cells suspension was stained with CD31-PE (Miltenyi Biotec; 1:50), CD4 PB (eBioscience 1:100), CD8 APC-Cy7 (eBioscience 1:100), CD45 APC (eBioscience 1:100), CD11b FITC (eBioscience 1:100), F4/80 Pe VIO770 Vio770 (Miltenyi Biotec; 1:50). The single cells suspension was also blocked with FC receptor blocker (Innovex NB335) as the manufactory instruction suggests. Cells were sorted using the BD FACSAria Fusion (Becton Dickinson) and analyzed at BD FACSCANTO II (Becton Dickinson). Cells data were analyzed using the FlowJo software.

### Cell Culture

Human primary muscle cells from healthy donors and FSHD patients were cultured in cell culture incubator at 37°C, 5% CO2 and 5% O2. The cells growing in proliferating media Ham's F-10 supplemented with 1X L-Glutamine, 20% FBS, 1% Pen/Strep, 10 ng/ml bFGF and 1 µM dexamethasone. The day after the growth medium was replaced by differentiation media (DMEM:F12 supplemented with 20% knockout serum replacement with 3.151 g/l glucose, 10 mM MEM non-essential amino acids, and 100 mM sodium pyruvate). Cells were harvested 96h after induction of differentiation.

### Transfection and transduction

DUX4 and non-silencing control siRNAs were purchased from Dharmacon and used at a final concentration of 25 nM. Transfection of siRNAs was performed by using Lipofectamine 3000 Transfection Reagent following manufacturer's instructions.

### Histological analysis

For hematoxylin and eosin staining, frozen sections were washed with PBS 1X to rehydrate them, fixed in 4% PFA for 10 min, and washed in PBS 1X and briefly in distilled water. Then, sections were stained in hematoxylin (100%), washed in running tap water, and then counterstained with eosin (100%). After rinsing in distilled water, cryosections were dehydrated with increasing percentages of ethanol, fixed in o-Xylene, and mounted. The OIL RED O staining (Bio-optica 30-30112) was performed following manufacturer's instructions.

### Immunofluorescence analysis

Muscle tissues were collected and snap-frozen in liquid nitrogen-cooled isopentane and stored at -80 °C until sectioning. Serial 10 µm transverse cryosections were cut from skeletal muscle (Tibialis Anterior). Tissue sections were fixed in 4% PFA for 10 min, and permeabilized with 100% methanol for 6 min at -20°C. Muscle sections were blocked with 5% BSA in PBS 1X for 1 hour at room temperature, to avoid nonspecific binding. Immunostaining with primary antibodies was performed overnight at 4°C. Specifically the slides were stained with Laminin (1:300), Collagen (COL1a-1:250), Perilipin (1:250), dsRNA (K1-1:100); all the antibodies were diluted in the blocking solution. For F4/80 and PDGFRα staining, the sections were fixed in 4% PFA for 10 min, and permeabilized with Triton X-100 0,25% in PBS 1X for 20 min at RT and blocked with 5% BSA in PBS 1X for 1 hour at RT. The primary antibodies immunostaining was performed overnight at 4°C, the antibodies were diluted 1:100 in the blocking solution. The day after, was performed 3 washes in PBS 1X and the tissues were treated with corresponding secondary antibodies in Alexa Fluor 488 or Alexa Fluor 555, at a dilution of 1:500 for 1 h at room temperature. After 3 washes for 10 min at RT, sections were stained with DAPI 1:1000 in PBS 1X for 5 min at RT, washed and mounted and air-dried before imaging using microscopy.

### Imaging and quantification

Images were collected using an AxioImage M2 fluorescent microscope (Zeiss, Germany). CSA was manually calculated using Imaged based on the Laminin staining. The areas were measured on images taken randomly to cover the entire transverse area of the muscle section. The Oil Red O area, were quantified using Imaged, calculating the area of the red pixels per field. The same procedure has been applied to evaluate the content of Collagen positive areas.

### RNA extraction and RT-qPCR

Total RNA from isolated muscles was extracted with TRI Reagent (Invitrogen) using the manufacturer's instructions. The RNA extraction from cells was performed using PureLink RNA Mini Kit (ThermoFisher Scientific, #12183025), following manufacturer's instructions. In details, cells from a well of a 6-well plate are lysed in 300 µl of Lysis buffer supplemented with 2-mercaptoethanol and homogenized through a 21-gauge syringe needle. One volume of 70% ethanol was added and the lysate was loaded onto the spin cartridge provided by the kit. Samples were washed with 350 µl of wash buffer I, centrifuged at 12,000 g for 15 seconds at RT and then on-column DNAseI treatment was performed (PureLink DNase Set, ThermoFisher Scientific, #12185010) for 20 min at RT, and then a second wash with 350 µl of wash buffer I was performed. From now on, the steps indicated in the manufacturer's datasheet (from step 8 to the end) were performed and RNA was eluted in 30 µl of RNAse-free water. The RNA obtained from cells or muscles were quantified by nanodrop and stored at -80°C. For cDNA synthesis, SuperScript^{™} III First-Strand Synthesis SuperMix for qRT-PCR (Thermo Fisher Scientific, #11752250) was used, using between 500 ng and 1 ug of RNA as input, following manufacturer's instructions. Quantitative real-time PCR was performed with the iTaq Universal SYBR Green Supermix (Bio-Rad,1725122). Oligonucleotide primer sequences was reported in Table 1.

**TABLE 1: Primers List**

| | | |
|---|---|---|
| CTTCCGTGAAATTCTGGCTGAATG | SEQ ID no. 1 | hDUX4 dual Fw |
| TTTTTTTTTTTTTTTTTCTATAGGATCCACAGG | SEQ ID no. 2 | hDUX4 dual Rv |
| TTGGTGATTCCACTGGATTTCT | SEQ ID no. 3 | hHSATII Fw |
| TCGGATGGAATCAATGAAGGGA | SEQ ID no. 4 | hHSATII Rev |
| ATTGGCAACACCGTATTCTGCT | SEQ ID no. 5 | hHERV-K Fw |
| CAGTCAAAATATGGACGGATGGT | SEQ ID no. 6 | hHERV-K Rev |
| ATATCCTGCCTGGATGGGGT | SEQ ID no. 7 | hERVL Fw |
| GAGCTTCTTAGTCCTCCTGTGT | SEQ ID no. 8 | hERVL Rev |
| TGGTTGGGCCACCTAGAAGTA | SEQ ID no. 9 | hTLR3 Fw |
| TCTCCATTCCTGGCCTGTG | SEQ ID no. 10 | hTLR3 Rev |
| GAGCAACTTCTTTCAACCACAG | SEQ ID no. 11 | h MDA5 Fw |
| CACTTCCTTCTGCCAAACTTG | SEQ ID no. 12 | hMDA5 Rev |
| CCAGCATTACTAGTCAGAAGGAA | SEQ ID no. 13 | hRIG-I Fw |
| CACAGTGCAATCTTGTCATCC | SEQ ID no. 14 | hRIG-I Rev |
| GGGAGAGATTGACCAGCAGT | SEQ ID no. 15 | hSTAT-3 Fw |
| TGATTCTTCCCACAGGCACC | SEQ ID no. 16 | hSTAT-3 Rev |
| CATTCAGATGTAGCGGATAATGG | SEQ ID no. 17 | hIFN-*γ* Fw |
| CACTCTTTTGGATGCTCTGGT | SEQ ID no. 18 | hIFN-*γ* Rev |
| CTTGGATCAGCTGCAGAACT | SEQ ID no. 19 | hSTAT-1 Fw |
| GTGCGGTCCCATAACACTTG | SEQ ID no. 20 | hSTAT-1 Rev |
| CCAGAACAGATTTGAGAG | SEQ ID no. 21 | hIL-6 FW |
| CTACATTTGCCGAAGAGC | SEQ ID no. 22 | hIL-6 Rev |
| AGTTTGAGGTGAGACGGCA | SEQ ID no. 23 | mmIFN-*γ*Fw |
| TCCCAATCTGTCTGCAGTGG | SEQ ID no. 24 | mmIFN-*γ* Rev |
| ATGACCTCCTCTCACAGCTG | SEQ ID no. 25 | mmSTAT*-*1 Fw |
| CCTTTCTTCCTTCAGACAGTTGT | SEQ ID no. 26 | mmSTAT-1 Rev |
| GCAATATAGCCGATTCCTGCA | SEQ ID no. 27 | mmSTAT-3 FW |
| TCTGGAGGAGGCGAGACT | SEQ ID no. 28 | mmSTAT-3 Rev |
| AGCTGCAGGTCCAAGAGCG | SEQ ID no. 29 | mmIL-28B Fw |
| GGTGGTCAGGGCTGAGTCATT | SEQ ID no. 30 | mmIL-28B Rev |
| GGTGTCCGTGACTAACTCCAT | SEQ ID no. 31 | mmISG15 Fw |
| TGGAAAGGGTAAGACCGTCCT | SEQ ID no. 32 | mmISG15 Rev |
| CAGGAGCTGTACGGCTTCC | SEQ ID no. 33 | mmOASL Fw |
| CCTACCTTGAGTACCTTGAGCAC | SEQ ID no. 34 | mmOASL Rev |
| GGAGAGGAGACTTCACAGAGG | SEQ ID no. 35 | mmIL-6 Fw |
| CCAGTTTGGTAGCATCCATC | SEQ ID no. 36 | mmIL-6 Rev |
| ACCACAATGGTGCTGTTGAA | SEQ ID no. 37 | mmPDGFRα Fw |
| AATCTCTGGGGCAAAGGTCT | SEQ ID no. 38 | mmPDGFRα Rev |
| CCCCACAAGGCTAGTCTCAT | SEQ ID no. 39 | mmIAPEZ-int Fw |
| GTCCCGTGTCGTTTTACCTG | SEQ ID no. 40 | mmIAPEZ-int Rev |
| AGACATTCCTGAGGCTGCTT | SEQ ID no. 41 | mmRLTR1B Fw |
| AGGAGACAGTGGATTCGACC | SEQ ID no. 42 | mmRLTR1B Rev |
| CTTGCCCTTAAAGGTCTAAAAGCA | SEQ ID no. 43 | mmIAP Pol Fw |
| GCGGTATAAGGTACAATTAAAAGATATGG | SEQ ID no. 44 | mmIAP Pol Rev |
| GGCCTGGACGACAAAACC | SEQ ID no. 45 | mmPerilipin Fw |
| CAGGATGGGCTCCATGAC | SEQ ID no. 46 | mmREV Perilipin Rev |
| CCTCAGGGTATTGCTGGACA | SEQ ID no. 47 | mmCol1a1 Fw |
| GAAGGACCTTGTTTGCCAGG | SEQ ID no. 48 | mmCollalmm Rev |

### RESULTS

### DUX4 activates a viral mimicry response in FSHD muscle cells

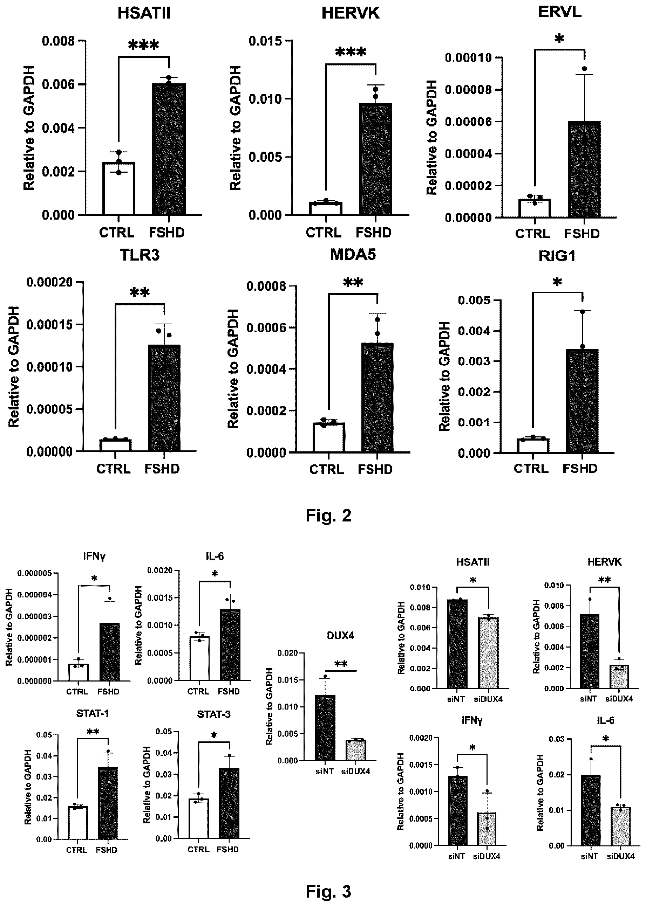

Among direct DUX4 targets in human and mouse are endogenous retroviruses and other repetitive sequences ^{4,56}. Activation of these sequences by DUX4 leads to the accumulation of double strand RNA (dsRNA), which significantly contributes to DUX4 toxicity ^{57,58}. In other contexts, growing evidence indicates that an important consequence of repeats activation and dsRNA production is the induction of a "viral mimicry" status encompassing innate and adaptive immune responses culminating with the activation of interferon gamma (IFN-γ) signaling and a STAT-linked IFN response ⁵⁹. This pathway is initiated upon recognition of repeat-derived dsRNA by cytosolic RNA sensors such as MDA5, RIG1 or TLR3 (**Figure 2**). Whether a pro-inflammatory viral mimicry pathway is active in FSHD is currently unknown. Intriguingly, the inventors found a significant increase in the expression of direct DUX4 target repeat RNAs and RNA sensors in primary muscle cells of FSHD patients compared to healthy donor muscle cells (**Figure 2**).

Upon IFN binding, IFNGR1 intracellular domain opens out to allow association of downstream signaling components JAK2, JAK1 and STAT-1, leading to STAT-1 activation, nuclear translocation, and transcription of IFN-regulated genes ⁶⁰. Notably, it was found a significant IFN-γ, IL-6, STAT-1 and STAT-3 activation in FSHD compared to healthy donor primary muscle cells (**Figure 3**).

To determine if DUX4, through the induction of repeats, leads to activation of the above pathway, the inventors silenced DUX4 in primary muscle cells of FSHD. Intriguingly, a significant decrease in repeat RNA, IFN-γ and IL-6 levels was found in primary FSHD muscle cells knockdown for DUX4 compared to control knockdown cells (**Figure 3**).

### Treatment with recombinant IFN-γ is sufficient to induce the pathway in healthy muscle cells

Based on the data above, to investigate the relevance and the importance of IFN-γ, primary muscle cells from healthy donors were treated with recombinant IFN-γ. Interestingly, it was found that IFN-γ treatment is sufficient to activate the pro-inflammatory viral mimicry pathway in healthy donor muscle cells (**Figure 4**).

### Activation of the IFN-γ response signature selectively in inflamed FSHD muscle

To independently support the above findings, the inventors took advantage of publicly-available gene expression datasets using muscle biopsies from healthy donors, FSHD patients and patients affected by other muscular dystrophies. Gene set enrichment analysis (GSEA) was performed on the list of genes significantly upregulated in: i. FSHD patients compared to healthy donors; ii. FSHD patients compared to patients affected by another muscular dystrophy; and iii. inflamed compared to non-inflamed muscles of FSHD patients. Notably, for all comparisons a significant activation of the IFN-γ response signature was found (**Figure 5**). Collectively, these results indicate that DUX4 induces the viral mimicry pathway, leading to activation of an IFN-y/IL-6/STAT inflammatory response in FSHD.

### Increased repeats, dsRNA and inflammatory factors in FLExDUX4 mice

To evaluate the relevance of their findings in vivo, the inventors took advantage of the FLExDUX4 mouse model of FSHD. This is a transgenic mouse expressing human DUX4 in an inducible and skeletal muscle fiber-restricted manner ³⁰. The model shows DUX4 dose-dependent myopathy characterized by reductions in muscle size and force-generation capacity. Histological analysis shows muscle fiber size heterogeneity, fiber necrosis and central nucleation, mononuclear cell infiltrate, and fibrosis. The FLExDUX4 model is the most widely used FSHD animal model in academia ^{31,61-68} and is the only one used by companies developing FSHD therapeutics. Thanks to the skeletal muscle fiber-specific and tamoxifen inducible ACTA1-MCM driver, in this model DUX4 expression is conditional, titratable, and restricted to myofibers, thus the time of onset and the severity of phenotypes can be both controlled to mimic different aspects of the human disease. For example, a chronic model (23 weeks old FLExDUX4 mice) expressing constitutively low DUX4 levels when not induced mimics the slow progression of the human disease ³⁰.

By comparing muscle RNA extracted from control (ACTA1-MCM) and FLExDUX4 mice treated with tamoxifen, the inventors found a significant upregulation of direct DUX4 repeat targets and accumulation of dsRNA in FLExDUX4 compared to control mice (**Figure 6**). This was associated to a significant increase in the expression of IFN-γ, IL-6 and STAT factors in FLExDUX4 mice compared to control mice (**Figure 7**). Increased IFN-γ expression was confirmed also at protein level (**Figure 8**).

### Fibro-Adipogenic Progenitor expansion in FLExDUX4 mice

Expansion of Fibro-Adipogenic Progenitors (FAPs) in FSHD patients and another mouse model of FSHD has been reported ^{11,11,69,70}, which might contribute to muscle wasting and its fibro-fatty substitution. Intriguingly, it was found a significant expansion of FAPs and their fibro-fatty activation in muscle of FLExDUX4 mice compared to the ACTA1-MCM control (**Figure 9**).

### FAPs are the first extra-muscular cells affected by DUX4 expression

Using FACS sorting, different mononuclear cells composing skeletal muscle were isolated from FLExDUX4 and its relative control at 2-, 6- and 15-days post tamoxifen injection (d.p.i.). Intriguingly, it was found that FAPs are the first extra-muscular cell type affected by DUX4 induction being significantly increased in FLExDUX4 compared to control mice already at the earlier timepoint analyzed. Macrophages were significantly increased only starting from 6 d.p.i, while CD4 and CD8 lymphocytes were not significantly increased in FLExDUX4 compared to control mice (**Figure 10**). Hence, among the extra-muscular cell types analyzed, FAPs are the first to be affected by DUX4 induction.

### IL-6 but not IFN-γ induction in FLExDUX4 FAPs

Given that they are the first extra-muscular cells affected by DUX4 expression, the expression of inflammatory molecules was monitored in FAPs. Interestingly, FAPs purified from induced FLExDUX4 muscle expressed significantly higher IL-6 levels compared to FAPs purified from control mice (**Figure 11**). On the contrary, no IFN-γ expression was detectable in FAPs purified from FLExDUX4 or control muscle (**Figure 11**). These results strongly suggest that IFN-γ is selectively expressed by FLExDUX4 muscle fibers.

### Confirmation of results in the chronic model of FSHD

As indicated above, FLExDUX4 mice can be used in an acute modality by inducing DUX4 expression with tamoxifen administration. Alternatively, since the system is leaky, FLExDUX4 mice express DUX4 constitutively at very low levels in the absence of tamoxifen administration. It has been reported that a chronic 23 weeks old FLExDUX4 mice model might mimic the slow progression of the human disease ³⁰. Importantly, a significant activation of inflammatory molecules, expansion and fibro-fatty activation of FAPs, and selective expression of IL-6 by FAPs were found in the chronic model of FSHD (**Figures 12-14**).

### Activation of the IFN-γ response signature in FLExDUX4 mice

To independently support the above findings, the inventors took advantage of publicly-available gene expression datasets using muscle biopsies from control ACTA1-MCM and FLExDUX4 mice treated with tamoxifen. GSEA was performed on the list of genes significantly upregulated in FLExDUX4 compared to control mice. Notably, a significant activation of the IFN-γ response signature was found in FLExDUX4 compared to control mice (Figure 15).

### Treatment with recombinant IFN-γ is sufficient to induce the pathway in healthy WT mice

Based on the data above, to investigate the relevance and the importance of IFN-γ, the inventors treated healthy WT mice with recombinant IFN-γ. Specifically, IFN-γ was injected in the tibialis anterior muscle of one leg, while the tibialis anterior of the contralateral leg was injected with vehicle (PBS), as control. Interestingly, IFN-γ treatment was sufficient to activate the pro-inflammatory viral mimicry pathway in healthy WT muscle (**Figure 16**).

Collectively, these results indicate that DUX4 induces the viral mimicry pathway, leading to activation of an IFN-y/IL-6/STAT inflammatory response in the FLExDUX4 model of FSHD.

### Therapeutic relevance of IFN-γ targeting

DUX4 was found to activate a proinflammatory pathway both in human and mice. Intriguingly, the data obtained in the invention indicate that IFN-γ is selectively produced by muscle fibers downstream of DUX4. Based on this, the inventors hypothesize that IFN-γ is on top of the pathogenic cascade leading to inflammation and muscle wasting in FSHD. Therefore, inhibiting IFN-γ should block the inflammation and significantly reduce disease severity. To test this, FLExDUX4 mice were treated with tamoxifen (to induce DUX4) and with an IFN-γ neutralizing antibody, or an isotype control antibody (**Figure 17**). Safety and efficacy of the treatment was evaluated at histological, cellular, and molecular levels. In this experiment, IFN-γ inhibition decreased expression of IFN-response genes and lead to a significant amelioration of the disease in FLExDUX4 mice at histological level (**Figure 16**). IFN-γ inhibition also caused a significant reduction of FAPs and macrophages, and a significant reduction of fibro-fatty muscle tissue substitution (**Figure 18**). These beneficial effects of IFN-γ inhibition were associated also to a significant preservation of muscle size (**Figure 18**).

Collectively, these results indicate that inhibition of IFN-γ signaling significantly reduce muscle inflammation, FAPs expansion, fibro-fatty conversion, and overall disease progression in the mouse model of FSHD.

All in all, the present results indicate that DUX4 induces the production of dsRNA in muscle fibers, which activate the viral mimicry pathway leading to the production of IFN-γ and a first wave of IL-6 from skeletal muscle fibers. Next, IL-6 activates FAPs which proliferate, produce additional IL-6, and differentiate in fibroblasts and adipocyte. Altogether, this inflammatory response leads to muscle fiber degeneration and its fibro-fatty substitution (**Figure 19**). Importantly, the present invention demonstrates that this vicious cycle can be blocked by IFN-γ inhibition and, by disclosing a molecular mechanism for the inflammatory insult caused by aberrant DUX4 expression, identifies a novel therapeutic approach.

### REFERENCES

1. Mocciaro, E., Runfola, V., Ghezzi, P., Pannese, M. & Gabellini, D. DUX4 Role in Normal Physiology and in FSHD Muscular Dystrophy. Cells 10, (2021).
2. De Iaco, A. et al. DUX-family transcription factors regulate zygotic genome activation in placental mammals. Nat Genet 49, 941-945 (2017).
3. Whiddon, J. L., Langford, A. T., Wong, C. J., Zhong, J. W. & Tapscott, S. J. Conservation and innovation in the DUX4-family gene network. Nat Genet 49, 935-940 (2017).
4. Hendrickson, P. G. et al. Conserved roles of mouse DUX and human DUX4 in activating cleavage-stage genes and MERVL/HERVL retrotransposons. Nat Genet 49, 925-934 (2017).
5. Feng, Q. et al. A feedback loop between nonsense-mediated decay and the retrogene DUX4 in facioscapulohumeral muscular dystrophy. Elife 2015, (2015).
6. Campbell, A. E. et al. NuRD and CAF-1-mediated silencing of the D4Z4 array is modulated by DUX4-induced MBD3L proteins. Elife 7, (2018).
7. Resnick, R. et al. DUX4-Induced Histone Variants H3.X and H3.Y Mark DUX4 Target Genes for Expression. Cell Rep 29, 1812-1820.e5 (2019).
8. Jiang, S. et al. Single-nucleus RNA-seq identifies divergent populations of FSHD2 myotube nuclei. PLoS Genet 16, (2020).
9. Chau, J. et al. Relationship of DUX4 and target gene expression in FSHD myocytes. Hum Mutat 42, 421-433 (2021).
10. Brennan, C. M. et al. DUX4 expression activates JNK and p38 MAP kinases in myoblasts. Dis Model Mech 15, (2022).
11. Tawil, R. et al. A pilot trial of prednisone in facioscapulohumeral muscular dystrophy. FSH-DY Group. Neurology 48, 46-9 (1997).
12. Kissel, J. T. et al. Randomized, double-blind, placebo-controlled trial of albuterol in facioscapulohumeral dystrophy. Neurology 57, 1434-40 (2001).
13. Matsumura, T. et al. [A clinical trial of creatine monohydrate in muscular dystrophy patients]. Rinsho Shinkeigaku 44, 661-666 (2004).
14. Elsheikh, B. H. et al. Pilot trial of diltiazem in facioscapulohumeral muscular dystrophy. Neurology 68, 1428-9 (2007).
15. Van Der Kooi, E. L. et al. Effects of training and albuterol on pain and fatigue in facioscapulohumeral muscular dystrophy. J Neurol 254, 931-940 (2007).
16. Payan, C. A. et al. Periodic salbutamol in facioscapulohumeral muscular dystrophy: a randomized controlled trial. Arch Phys Med Rehabil 90, 1094-1101 (2009).
17. Wagner, K. R. et al. A phase I/IItrial of MYO-029 in adult subjects with muscular dystrophy. Ann Neurol 63, 561-571 (2008).
18. Passerieux, E. et al. Effects of vitamin C, vitamin E, zinc gluconate, and selenomethionine supplementation on muscle function and oxidative stress biomarkers in patients with facioscapulohumeral dystrophy: A double-blind randomized controlled clinical trial. Free Radic Biol Med 81, 158-169 (2015).
19. Sitzia, C. et al. Preliminary evidences of safety and efficacy of flavonoids- And omega 3-based compound for muscular dystrophies treatment: A randomized double-blind placebo controlled pilot clinical trial. Front Neurol 10, (2019).
20. Mellion, M. L. et al. Phase 1 clinical trial of losmapimod in facioscapulohumeral dystrophy: Safety, tolerability, pharmacokinetics, and target engagement. Br J Clin Pharmacol 87, 4658-4669 (2021).
21. Statland, J. M. et al. Randomized phase 2 study of ACE-083, a muscle-promoting agent, in facioscapulohumeral muscular dystrophy. Muscle Nerve 66, 50-62 (2022).
22. Kissel, J. T. et al. Pilot trial of albuterol in facioscapulohumeral muscular dystrophy. Neurology 50, 1402-1406 (1998).
23. Frisullo, G. et al. CD8+ T cells in facioscapulohumeral muscular dystrophy patients with inflammatory features at muscle MRI. J Clin Immunol 31, (2011).
24. Janssen, B. H. et al. Distinct disease phases in muscles of facioscapulohumeral dystrophy patients identified by MR detected fat infiltration. PLoS One 9, (2014).
25. Dahlqvist, J. R. et al. Relationship between muscle inflammation and fat replacement assessed by MRI in facioscapulohumeral muscular dystrophy. J Neurol 266, 1127-1135 (2019).
26. Dahlqvist, J. R. et al. Evaluation of inflammatory lesions over 2 years in facioscapulohumeral muscular dystrophy. Neurology 95, E1211-E1221 (2020).
27. Wong, C. J. et al. Regional and bilateral MRI and gene signatures in facioscapulohumeral dystrophy: implications for clinical trial design and mechanisms of disease progression. Hum Mol Genet 33, 698-708 (2024).
28. Wang, L. H. et al. MRI-informed muscle biopsies correlate MRI with pathology and DUX4 target gene expression in FSHD. Hum Mol Genet 28, 476-486 (2019).
29. Wong, C. J. et al. Longitudinal measures of RNA expression and disease activity in FSHD muscle biopsies. Hum Mol Genet 29, 1030-1044 (2020).
30. Jones, T. I. et al. Transgenic mice expressing tunable levels of DUX4 develop characteristic facioscapulohumeral muscular dystrophy-like pathophysiology ranging in severity. Skelet Muscle 10, (2020).
31. Gros, M. et al. Identification of Serum Interleukin 6 Levels as a Disease Severity Biomarker in Facioscapulohumeral Muscular Dystrophy. J Neuromuscul Dis 9, (2021).
32. Hunter, C. A. & Jones, S. A. IL-6 as a keystone cytokine in health and disease. Nature Immunology vol. 16 Preprint at https://doi.org/10.1038/ni.3153 (2015).
33. Pedersen, B. K. & Febbraio, M. A. Muscle as an endocrine organ: focus on muscle-derived interleukin-6. Physiol Rev 88, 1379-1406 (2008).
34. Pedersen, M. et al. Circulating levels of TNF-alpha and IL-6-relation to truncal fat mass and muscle mass in healthy elderly individuals and in patients with type-2 diabetes. in Mechanisms of Ageing and Development vol. 124 (2003).
35. Visser, M. et al. Relationship of interleukin-6 and tumor necrosis factor-alpha with muscle mass and muscle strength in elderly men and women: The health ABC study. Journals of Gerontology - Series A Biological Sciences and Medical Sciences 57, (2002).
36. Schaap, L. A., Pluijm, S. M. F., Deeg, D. J. H. & Visser, M. Inflammatory Markers and Loss of Muscle Mass (Sarcopenia) and Strength. American Journal of Medicine 119, (2006).
37. Taaffe, D. R., Harris, T. B., Ferrucci, L., Rowe, J. & Seeman, T. E. Cross-sectional and prospective relationships of interleukin-6 and c-reactive protein with physical performance in elderly persons: MacArthur studies of successful aging. Journals of Gerontology - Series A Biological Sciences and Medical Sciences 55, (2000).
38. Legrand, D. et al. Muscle strength and physical performance as predictors of mortality, hospitalization, and disability in the oldest old. J Am Geriatr Soc 62, (2014).
39. Haddad, F., Zaldivar, F., Cooper, D. M. & Adams, G. R. IL-6-induced skeletal muscle atrophy. J Appl Physiol 98, (2005).
40. Forcina, L., Franceschi, C. & Musarò, A. The hermetic and hermetic role of IL-6. Ageing Res Rev 80, (2022).
41. Pelosi, L. et al. Sustained systemic levels of IL-6 impinge early muscle growth and induce muscle atrophy and wasting in adulthood. Cells 10, (2021).
42. Joe, A. W. B. et al. Muscle injury activates resident fibro/adipogenic progenitors that facilitate myogenesis. Nat Cell Biol 12, (2010).
43. Wosczyna, M. N. et al. Mesenchymal Stromal Cells Are Required for Regeneration and Homeostatic Maintenance of Skeletal Muscle. Cell Rep 27, (2019).
44. Molina, T., Fabre, P. & Dumont, N. A. Fibro-adipogenic progenitors in skeletal muscle homeostasis, regeneration and diseases. Open Biol 11, (2021).
45. Uezumi, A., Fukada, S. I., Yamamoto, N., Takeda, S. & Tsuchida, K. Mesenchymal progenitors distinct from satellite cells contribute to ectopic fat cell formation in skeletal muscle. Nat Cell Biol 12, (2010).
46. Uezumi, A. et al. Fibrosis and adipogenesis originate from a common mesenchymal progenitor in skeletal muscle. J Cell Sci 124, (2011).
47. Mozzetta, C. et al. Fibroadipogenic progenitors mediate the ability of HDAC inhibitors to promote regeneration in dystrophic muscles of young, but not old Mdx mice. EMBO Mol Med 5, (2013).
48. Kopinke, D., Roberson, E. C., Correspondence, J. F. R. & Reiter, J. F. Ciliary Hedgehog Signaling Restricts Injury-Induced Adipogenesis In Brief Modulating ciliary Hedgehog signaling following muscle injury prevents fat deposition and improves muscle healing. Ciliary Hedgehog Signaling Restricts Injury-Induced Adipogenesis. Cell 170, (2017).
49. Lukjanenko, L. et al. Aging Disrupts Muscle Stem Cell Function by Impairing Matricellular WISP1 Secretion from Fibro-Adipogenic Progenitors. Cell Stem Cell 24, (2019).
50. Heredia, J. E. et al. Type 2 innate signals stimulate fibro/adipogenic progenitors to facilitate muscle regeneration. Cell 153, (2013).
51. Lemos, D. R. et al. Nilotinib reduces muscle fibrosis in chronic muscle injury by promoting TNF-mediated apoptosis of fibro/adipogenic progenitors. Nat Med 21, (2015).
52. Guadagnin, E., Mázala, D. & Chen, Y. W. STAT3 in Skeletal Muscle Function and Disorders. Int J Mol Sci 19, (2018).
53. Madaro, L. et al. Denervation-activated STAT3-IL-6 signalling in fibro-adipogenic progenitors promotes myofibres atrophy and fibrosis. Nat Cell Biol 20, (2018).
54. Bosnakovski, D. et al. Transcriptional and cytopathological hallmarks of FSHD in chronic DUX4-expressing mice. Journal of Clinical Investigation 130, (2020).
55. van den Heuvel, A. et al. Facioscapulohumeral dystrophy transcriptome signatures correlate with different stages of disease and are marked by different MRI biomarkers. Sci Rep 12, (2022).
56. Geng, L. N. et al. DUX4 Activates Germline Genes, Retroelements, and Immune Mediators: Implications for Facioscapulohumeral Dystrophy. Dev Cell 22, 38-51 (2012).
57. Shadle, S. C. et al. DUX4-induced dsRNA and MYC mRNA stabilization activate apoptotic pathways in human cell models of facioscapulohumeral dystrophy. PLoS Genet 13, e1006658 (2017).
58. Shadle, S. C. et al. DUX4-induced bidirectional HSATII satellite repeat transcripts form intranuclear double-stranded RNA foci in human cell models of FSHD. Hum Mol Genet 28, (2019).
59. Chen, R., Ishak, C. A. & Carvalho, D. D. de. Endogenous retroelements and the viral mimicry response in cancer therapy and cellular homeostasis. Cancer Discovery vol. 11 Preprint at https://doi.org/10.1158/2159-8290.CD-21-0506 (2021).
60. Mogensen, T. H. IRF and STAT transcription factors - From basic biology to roles in infection, protective immunity, and primary immunodeficiencies. Frontiers in Immunology vol. 10 Preprint at https://doi.org/10.3389/fimmu.2018.03047 (2019).
61. Lim, K. R. Q. et al. Inhibition of DUX4 expression with antisense LNA gapmers as a therapy for facioscapulohumeral muscular dystrophy. Proc Natl Acad Sci U S A 117, 16509-16515 (2020).
62. Bittel, A. J. et al. Membrane Repair Deficit in Facioscapulohumeral Muscular Dystrophy. Int J Mol Sci 21, 1-18 (2020).
63. Himeda, C. L., Jones, T. I. & Jones, P. L. Targeted epigenetic repression by CRISPR/dSaCas9 suppresses pathogenic DUX4-fl expression in FSHD. Mol Ther Methods Clin Dev 20, 298-311 (2020).
64. Bouwman, L. F. et al. Systemic delivery of a DUX4-targeting antisense oligonucleotide to treat facioscapulohumeral muscular dystrophy. Mol Ther Nucleic Acids 26, 813-827 (2021).
65. Lim, K. R. Q. et al. DUX4 Transcript Knockdown with Antisense 2'-O-Methoxyethyl Gapmers for the Treatment of Facioscapulohumeral Muscular Dystrophy. Mol Ther 29, 848-858 (2021).
66. Lu-Nguyen, N., Dickson, G., Malerba, A. & Popplewell, L. Long-Term Systemic Treatment of a Mouse Model Displaying Chronic FSHD-like Pathology with Antisense Therapeutics That Inhibit DUX4 Expression. Biomedicines 10, (2022).
67. Lu-Nguyen, N., Malerba, A., Herath, S., Dickson, G. & Popplewell, L. Systemic antisense therapeutics inhibiting DUX4 expression ameliorates FSHD-like pathology in an FSHD mouse model. Hum Mol Genet 30, (2021).
68. Lu-Nguyen, N., Malerba, A., Pineda, M. A., Dickson, G. & Popplewell, L. Improving Molecular and Histopathology in Diaphragm Muscle of the Double Transgenic ACTA1-MCM/FLExDUX4 Mouse Model of FSHD with Systemic Antisense Therapy. Hum Gene Ther 33, (2022).
69. Bosnakovski, D. et al. Persistent Fibroadipogenic Progenitor Expansion Following Transient DUX4 Expression Provokes a Profibrotic State in a Mouse Model for FSHD. Int J Mol Sci 23, (2022).
70. Di Pietro, L. et al. Non-myogenic mesenchymal cells contribute to muscle degeneration in facioscapulohumeral muscular dystrophy patients. Cell Death Dis 13, (2022).

## Claims

1. An inhibitor of the IFN-γ inflammatory pathway for use in the treatment and/or prevention of FSHD.

2. The inhibitor for use according to claim 1 being an inhibitor of IFN-γ expression and/or function.

3. The inhibitor for use according to claim 2 selected from the group consisting of:
a) polynucleotide capable of inhibiting the expression of IFN-γ or a polynucleotide coding for said polynucleotide;
b) a polypeptide, preferably an antibody or a synthetic or recombinant derivative thereof;
c) a polynucleotide coding for said polypeptide or a functional derivative thereof;
d) a small molecule.

4. The inhibitor for use according to claims 2 or 3, wherein the inhibitor of IFN-γ expression and/or function is anti-IFN-γ antibody.

5. The inhibitor for use according to claim 4 wherein the anti-IFN-γ antibody is selected from Emapalumab (Gamifant), fontolizumab (Huzaf), AMG 811, AB_2687717.

6. The inhibitor for use according to any one of claims 2-5 in combination with:
- an inhibitor of IL-6 expression and/or function; and/or
- an inhibitor of STAT-3 expression and/or function; and/or
- an inhibitor of JAK kinases expression and/or function.

7. The inhibitor for use according to claim 6 wherein the inhibitor of IL-6 expression and/or function and the inhibitor of STAT-3 expression and/or function and the inhibitor of JAK kinases expression and/or function are selected from the group consisting of:
a) polynucleotide capable of inhibiting the expression of the IL-6 and/or of STAT-3 and/or JAK kinases or a polynucleotide coding for said polynucleotide;
b) a polypeptide, preferably an antibody or a synthetic or recombinant derivative thereof;
c) a polynucleotide coding for said polypeptide or a functional derivative thereof;
d) a small molecule.

8. The inhibitor for use according to claims 6 or 7, wherein the inhibitor of IL-6 expression and/or function is anti-IL-6 antibody, preferably said anti IL-6 antibody is selected from Tocilizumab, Siltuximab, Sarilumab, ziltivekimab, olokizumab (CDP6038), elsilimomab, clazakizumab (BMS-945429, ALD518), sirukumab (CNTO 136), levilimab (BCD-089).

9. The inhibitor for use according to claims 6 or 7, wherein the inhibitor of STAT-3 expression and/or function is a small molecule inhibitor, preferably said small molecule inhibitor is selected from atovaquone, WP1066, TTI-101, VVD-130850, OPB-51602, silibinin e KT-333, or wherein the inhibitor of STAT3 expression and/or function is Danvatirsen.

10. The inhibitor of JAK kinases expression and/or function according to claims 6 or 7 wherein said inhibitor is selected from baricitinib (Olumiant), tofacitinib (Xeljanz), ruxolitinib (Jakavi, Opzelura), abrocitinib (Cibinqo), upadacitinib (Rinvoq), fedratinib (Inrebic).

11. The inhibitor for use according to any one of previous claims wherein the IFN-γ inflammatory pathway is initiated by overexpression of the *DUX4* gene.

12. The inhibitor for use according to any one of previous claims in combination with a further therapeutic agent and/or a therapeutic intervention.

13. A pharmaceutical composition for use in the treatment and/or prevention of FSHD said pharmaceutical composition comprising the inhibitor as defined in any one of previous claims and at least one pharmaceutical acceptable carrier and/or vehicle.

14. An in vitro method for evaluating the risk and/or for diagnosis and/or for prognosis and/or for monitoring progression and / or for monitoring the efficacy of a therapeutic treatment and/or for screening of FSHD in a subject or in an ex vivo or in vitro system, preferably an organoid such as artificial skeletal muscle, comprising the steps of:
a) detect or measure the amount or activity of the IFN-γ or of fragments thereof or of the polynucleotide encoding said protein or its fragments in an isolated biological sample obtained from the subject, which may be optionally comprised in an ex vivo or in vitro system, and
b) comparison with a proper control.
